(19) Europäisches Patentamt
European Patent Office
Office européen des brevets

(11) EP 4 722 365 A1

(12) EUROPEAN PATENT APPLICATION
published in accordance with Art. 153(4) EPC

(43) Date of publication:
**08.04.2026 Bulletin 2026/15**

(21) Application number: **24814348.9**

(22) Date of filing: **24.05.2024**

(51) International Patent Classification (IPC):
***C12N 15/113*** *(2010.01)* ***A61P 1/16*** *(2006.01)*
***A61K 31/713*** *(2006.01)* ***A61K 47/54*** *(2017.01)*
***A61K 31/712*** *(2006.01)* ***A61P 3/06*** *(2006.01)*

(52) Cooperative Patent Classification (CPC):
**A61K 31/712; A61K 31/713; A61K 47/54; A61P 1/16; A61P 3/06; C12N 15/113**

(86) International application number:
**PCT/CN2024/095285**

(87) International publication number:
**WO 2024/245151 (05.12.2024 Gazette 2024/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.05.2023 CN 202310609515**
**23.05.2024 CN 202410645783**

(71) Applicant: **Visirna Therapeutics (Suzhou) Co., Ltd.**
**Suzhou, Jiangsu 215028 (CN)**

(72) Inventors:
- **HE, Qionger**
  **Suzhou, Jiangsu 215028 (CN)**
- **WANG, Mingjie**
  **Suzhou, Jiangsu 215028 (CN)**
- **ZOU, Xiaoming**
  **Shanghai 200040 (CN)**
- **YANG, Hongkuan**
  **Shanghai 200040 (CN)**

(74) Representative: **Simmons & Simmons LLP (Munich)**
**Lehel Carré**
**Gewürzmühlstraße 11**
**80538 Munich (DE)**

Remarks:
The complete document including Reference Table(s) and the Sequence Listing(s) can be downloaded from the EPO website

# (54) DOUBLE-STRANDED NUCLEOTIDE COMPOUND FOR METABOLIC DISEASES AND USE THEREOF

(57) The present invention relates to the technical field of genetic engineering, and in particular, to a small interfering RNA for inhibiting the expression of diacylglycerol acyltransferase-2 (DGAT-2) and the use thereof in the preparation of a drug for treating diseases related to the expression of DGAT2.

EP 4 722 365 A1

## Description

### FIELD OF THE INVENTION

The present disclosure relates to the technical filed of genetic engineering, and in particular, to a small interfering RNA for inhibiting the expression of diacylglycerol acyltransferase-2 (DGAT-2) and the use thereof in the preparation of a drug for treating diseases related to the expression of DGAT2.

### BACKGROUND

RNA interference (RNAi) is a highly conserved phenomenon in the evolution process, in which highly efficient and specific degradation of a homologous mRNA is induced by a double-stranded RNA (dsRNA). The RNA molecule inhibits a biological process involving gene expression by destroying a specific mRNA. Because the RNAi technology can be used to specifically eliminate or shut down the expression of a specific gene, it has quickly become one of the research tools of greatest interest in the field of gene function and gene therapy research, and has been widely used in the field of gene function exploration and treatment of metabolic diseases, infectious diseases and malignant tumors.

Non-alcoholic fatty liver disease (NAFLD) is characterized by the accumulation of triglycerides in the patients' liver, which can develop into a severe form of non-alcoholic steatohepatitis (NASH) (Marchesini G, et al. Hepatology 2003; 37: 917-923).

Obesity and insulin resistance are common features shared in patients with NAFLD (Byrne CD and Targher G. J Hepatol 2015 Apr; 62(1S): S47-S64). With the increasing prevalence of obesity and type 2 diabetes, the incidence of NAFLD persistently increases and is estimated to reach 25% worldwide recently, where about 3-5% of the population suffers from NASH. NAFLD is the most common chronic liver disease at present, and NASH will become a main cause of liver transplantation in the United States in the next decade.

NAFLD has different characteristics in different stages, including simple hepatic steatosis, and more serious NASH in which steatosis is accompanied by lobular inflammation and ballooning degeneration, and hepatocyte degeneration. NASH is a progressive disease, which may lead to cirrhosis and hepatocellular carcinoma (Farrell GC and Larter CZ. Hepatology 2006; 43: S99-S112; Cohen JC et al., Science 2011; 332: 1519-1523). The risk of development into advanced liver disease depends on factors such as the severity of inflammation and fibrosis and the presence of other diseases, such as obesity, type 2 diabetes and high cholesterol.

Diacylglycerol acyltransferase (DGAT) is a key enzyme in the synthesis of triglyceride (TG). DGAT catalyzes the last step of TG synthesis by linking sn-1,2-diacylglycerol (DAG) to acyl coenzyme A.

Although the subtypes DGAT1 and DGAT2 have different functions and tissue expression patterns, they both have important functions in TG synthesis. DGAT1 mainly exists in small intestine and white adipose tissue (WAT), while DGAT2 mainly exists in liver and WAT, (Cases S, et al. Proc Natl Acad Sci USA 1998; 95, 13018-13023; Cases S, et al. J Biol Chem 2001; 276, 38870-38876). DGAT1 and DGAT2 (Cao J, et al., J Lipid Res 2007; 48: 583-591; Cheng D, et al., J Biol Chem 2008; 283: 29802-29811), subcellular localization (Stone SJ, et al., J Biol Chem 2004; 279: 11767-11776), and their physiological regulatory functions are different (Meegalla RL, et al. Biochem Biophys Res Commun 2002, 298, 317-323). Inhibition of DGAT2 by treatment with an anti-sense oligonucleotide is shown to improve hepatic steatosis and blood lipid levels in several studies in rodent models of obesity, and the data shows that these effects are associated with the decrease of hepatic lipid synthesis (Yu XX, et al., Hepatology 2005; 42:362-371, and Yamaguchi K et al. HEPATOLOGY, Vol. 45, No. 6, 2007 1366-1374). A recent academic paper also reported the long-term inhibition of DGAT2 mRNA and the significant improvement of fatty liver phenotype (Yenilmez, B et al., Molecular Therapy Vol. 30 No 3 March 2022).

The inhibition of DGAT2 may also lead to the increase of fat oxidation, because DGAT2 ASO reduces the expression of lipogenic genes: ACC1, mtGPAT, SCD1, and SREBP1, and increases the expression of oxidation/thermogenic genes CPT1 and UCP2 (Yu XX, et al. Hepatology 2005; 42: 362-371).

These studies indicate that DGAT2 inhibition may be a feasible strategy to develop treatments for NAFLD/NASH and other metabolic syndromes. By reducing triglycerides, improving insulin sensitivity and reducing hepatic steatosis, targeted DGAT2 inhibition may provide therapeutic benefits.

Small molecule inhibitors and ASOs targeting DGAT2 are reported to have promising results in reducing hepatic steatosis in clinical trials. siRNA technology has been proved to have significant advantages over traditional methods in terms of improved specificity, durability and safety. Targeting DGAT2 with siRNA may reduce hepatic steatosis and improve the metabolic parameters in patients with NAFLD/NASH and other metabolic disorders.

### SUMMARY OF THE INVENTION

The present invention aims to discover and develop a siRNA that can inhibit and/or reduce DGAT2 expression and its derivatives.

In a first aspect of the present invention, a siRNA is provided, which comprises a sense strand and an anti-sense strand, where the sense strand and the anti-sense strand include a duplex region formed by reverse complementarity. In some embodiments according to the first aspect of the present invention, at least part of the sense strand and at least part of the anti-sense strand are reversely complementary, to form a duplex region. In some embodiments according to the first aspect of the present invention, the length of the duplex region is 17 to 22 base pairs. In some embodiments according to the first aspect of the present invention, the length of the duplex region is 21 base pairs.

In some embodiments according to the first aspect of the present invention, the sense strand of the siRNA comprises any one of the sequences set forth in SEQ ID NOs: 1-96. In some embodiments according to the first aspect of the present invention, the sense strand of the siRNA comprises 17 to 22 consecutive bases of any one of the sequences set forth in SEQ ID NOs: 1-96. In some embodiments according to the first aspect of the present invention, the sense strand of the siRNA comprises 17, 18, 19, 20, 21 or 22 consecutive bases of any one of the sequences set forth in SEQ ID NOs: 1-96. In some embodiments according to the first aspect of the present invention, the sense strand of the siRNA comprises 20 consecutive bases of any one of the sequences set forth in SEQ ID NOs: 1-96.

In some embodiments according to the first aspect of the present invention, the anti-sense strand of the siRNA comprises any one of the nucleotide sequences set forth in SEQ ID NOs: 97-192. In some embodiments according to the first aspect of the present invention, the anti-sense strand of the siRNA comprises 17 to 22 consecutive bases of any one of the sequences set forth in SEQ ID NOs: 97-192 and 194-199. In some embodiments according to the first aspect of the present invention, the anti-sense strand of the siRNA comprises 17, 18, 19, 20, 21 or 22 consecutive bases of any one of the sequences set forth in SEQ ID NOs: 97-192 and 194-199. In some embodiments according to the first aspect of the present invention, the anti-sense strand of the siRNA comprises 20 consecutive bases of any one of the sequences set forth in SEQ ID NOs: 97-192 and 194-199.

In some embodiments according to the first aspect of the present invention, the sense strand of the siRNA comprises 17 to 22 consecutive bases of any one of the sequences set forth in SEQ ID NOs: 1-96, the anti-sense strand of the siRNA comprises 17 to 22 consecutive bases of any one of the sequences set forth in SEQ ID NO: 97-192, at least part of the sense strand and at least part of the anti-sense strand are reversely complementary to form a duplex region, and the length of the duplex region is 17 to 22 base pairs.

In some embodiments according to the first aspect of the present invention, the sense strand of the siRNA comprises 17 to 22 (and further 20) consecutive bases of a sequence as set forth in SEQ ID NO: 77 or SEQ ID NO: 201, and the anti-sense strand comprises 17 to 22 (and further 20) consecutive bases of a sequence as set forth in SEQ ID NO: 173; or the sense strand comprises 17 to 22 (and further 20) consecutive bases of a sequence as set forth in SEQ ID NO: 17, and the anti-sense strand comprises 17 to 22 (and further 20) consecutive bases of a sequence as set forth in SEQ ID NO: 113; or the sense strand comprises 17 to 22 (and further 20) consecutive bases of a sequence as set forth in SEQ ID NO: 80, and the anti-sense strand comprises 17 to 22 (and further 20) consecutive bases of a sequence as set forth in SEQ ID NO: 176.

In some embodiments, the sense strand of the siRNA comprises a sequence as set forth in SEQ ID NO: 77 or SEQ ID NO: 201, and the anti-sense strand comprises 20 consecutive bases of a sequence as set forth in SEQ ID NO: 173, 194, or 195. In some embodiments, the sense strand of the siRNA comprises a sequence as set forth in SEQ ID NO: 77, and the anti-sense strand comprises AAGCAAAUAGUCUAUGGUGU (SEQ ID NO: 194). In some embodiments, the sense strand of the siRNA comprises a sequence as set forth in SEQ ID NO: 77, and the anti-sense strand comprises UAAGCAAAUAGUCUAUGGUGU (SEQ ID NO: 195).

In some embodiments, the sense strand of the siRNA comprises a sequence as set forth in SEQ ID NO: 17, and the anti-sense strand comprises 20 consecutive bases in a sequence as set forth in SEQ ID NO: 113, 196, or 197. In some embodiments, the sense strand of the siRNA comprises a sequence as set forth in SEQ ID NO: 17, and the anti-sense strand comprises UUAAAUAACCCACAGACACC (SEQ ID NO: 196). In some embodiments, the sense strand of the siRNA comprises a sequence as set forth in SEQ ID NO: 17, and the anti-sense strand comprises UUUAAAUAACCCACAGACACC (SEQ ID NO: 197).

In some embodiments, the sense strand of the siRNA comprises a sequence as set forth in SEQ ID NO: 80, and the anti-sense strand comprises 20 consecutive bases of a sequence as set forth in SEQ ID NO: 176, 198 or 199. In some embodiments, the sense strand of the siRNA comprises a sequence as set forth in SEQ ID NO: 80, and the anti-sense strand comprises UGUAUUUCUGGAACUUCUUC (SEQ ID NO: 198). In some embodiments, the sense strand of the siRNA comprises a sequence as set forth in SEQ ID NO: 80, and the anti-sense strand comprises UUGUAUUUCUGGAACUUCUUC (SEQ ID NO: 199).

In some embodiments, the sense strand of the siRNA comprises a sequence as set forth in SEQ ID NO: 77 or 201, and the anti-sense strand comprises 20 consecutive bases of a sequence as set forth in SEQ ID NO: 173, 194, or 200. In some embodiments, the sense strand of the siRNA comprises a sequence as set forth in SEQ ID NO: 77, and the anti-sense strand comprises AAGCAAAUAGUCUAUGGUGU (SEQ ID NO: 194). In some embodiments, the sense strand of the siRNA comprises a sequence as set forth in SEQ ID NO: 77, and the anti-sense strand comprises AAAGCAAAUAGUCUAUGGUGU (SEQ ID NO: 200). In some embodiments, the sense strand of the siRNA comprises a sequence as set forth in SEQ ID NO: 201, and the anti-sense strand comprises a sequence as set forth in SEQ ID NO: 200.

In some embodiments according to the first aspect of the present invention, the sense strand of the siRNA comprises the nucleotide sequence of the sense strand of one siRNA molecule as shown in Table 2, and the anti-sense strand comprises the nucleotide sequence of the anti-sense strand of the same siRNA molecule as shown in Table 2. In certain embodiments, the siRNA comprises one or more modified nucleotide(s), which can be located at any suitable position(s) in the sense strand and/or the anti-sense strand of the siRNA. In certain embodiments, the siRNA comprises one or more modified nucleotides at modified nucleotide position(s) in the corresponding siRNA molecule in Table 2. In certain embodiments, the siRNA has the same nucleotide sequence as the corresponding siRNA molecule in Table 2, and has modified nucleotide(s) at the same position(s). In certain embodiments, the siRNA has the same nucleotide sequence as the corresponding siRNA molecule in Table 2, and has the same modified nucleotide(s) at the same position(s).

In some embodiments, the siRNA comprises :

a) a sense strand comprising the following nucleotide sequence (5'→3'): ggugucugUfGfGfguuauuuaaa, and an anti-sense strand comprising the following nucleotide sequence (5'→3'): u*Uf*u*AfaAfuaaccCfaCfaGfaCfaCf*c,
b) a sense strand comprising the following nucleotide sequence: gaagaaguUfCfCfagaaauacaa, and an anti-sense strand comprising the following nucleotide sequence (5'→3'): u*Uf*g*UfaUfuucugGfaAfcUfuCfuUf*c;
c) a sense strand comprising the following nucleotide sequence: acaccauaGfAfCfuauuugcuua, and an anti-sense strand comprising the following nucleotide sequence (5'→3'): u*Af*a*GfcAfaauagUfcUfaUfgGfuGf*u;
d) a sense strand comprising the following nucleotide sequence: acaccauaGfAfCfuauuugcuuu, and an anti-sense strand comprising the following nucleotide sequence (5'→3'): a*Af*a*GfcAfaauagUfcUfaUfgGfuGf*u;

where * indicates that two nucleotides are connected by a phosphorothioate linkage, lowercase letters (*e.g.*, a, u, g, and c) indicate that the ribose of the nucleotide is modified with 2'-methoxy (*i.e.,* 2'-O-methyl), and capital letters A, U, C, G and T followed by "f" indicate that the ribose of the nucleotide is modified with 2'-fluoro.

In some embodiments, the siRNA further comprises, at the 3' and/or 5' ends of the sense strand or anti-sense strand, an abasic nucleotide (optionally an inverted abasic nucleotide). In some embodiments, the abasic nucleotide or the inverted abasic nucleotide is connected to the 3' and/or 5' ends of the sense strand or the anti-sense strand by a phosphorothioate linkage. In some embodiments, the siRNA further comprises a 5'-terminal vinyl phosphonate (VP) modification at the 5' end of the anti-sense strand. In some embodiments, the VP modification is connected to the 5' end of the anti-sense strand by a phosphorothioate linkage.

In some embodiments, the siRNA comprises:

a) a sense strand comprising the following nucleotide sequence (5'→3'): (invAb)*ggugucugUfGfGfguuauuuaaa*(invAb), and an anti-sense strand comprising the following nucleotide sequence (5'→3'): u*Uf*u*AfaAfuaaccCfaCfaGfaCfaCf*c,
b) a sense strand comprising the following nucleotide sequence: (invAb)*gaagaaguUfCfCfagaaauacaa, and an anti-sense strand comprising the following nucleotide sequence (5'→3'): u*Uf*g*UtaUfuucugGfaAfcUtuCtuUt*c or Vpu*Uf*g*UfaUfuucugGfaAfcUfuCfuUf*c;
c) a sense strand comprising the following nucleotide sequence: (invAb)*acaccauaGfAfCfuauuugcuua, and an anti-sense strand comprising the following nucleotide sequence (5'→3'): u*Af*a*GfcAfaauagUfcUfaUfgGfuGf*u or Vpu*Af*a*GfcAfaauagUfcUfaUfgGfuGf*u;
d) a sense strand comprising the following nucleotide sequence: (invAb)*acaccauaGfAfCfuauuugcuuu*(invAb), and an anti-sense strand comprising the following nucleotide sequence (5'→3'): a*Af*a*GfcAfaauagUfcUfaUfgGfuGf*u;

where * indicates that two nucleotides are connected by a phosphorothioate linkage, lowercase letters (*e.g.*, a, u, g, and c) indicate that the ribose of the nucleotide is modified with 2'-methoxy (*i.e.,* 2'-O-methyl), and capital letters A, U, C, or G and T followed by "f" indicate that the ribose of the nucleotide is modified with 2'-fluoro, invAb represents an inverted abasic nucleotide, and VP represents vinyl phosphonate.

In some embodiments, the siRNA further comprises a ligand group at the 3' and/or 5' ends of the sense strand. Any suitable ligand group can be used. In some embodiments, the siRNA further comprises, at the 3' and/or 5' ends of the sense strand, a ligand group provided in the present application, for example, but not limited to, L96 or NAG37. In some embodiments, the siRNA further comprises, at the 3' end of the sense strand, a ligand group provided in the present application, for example, but not limited to, L96 or NAG37.

In some embodiments according to the first aspect of the present invention, the siRNA is selected from dS004 - dS099, as set forth in Table 2. In some embodiments according to the first aspect of the present invention, the siRNA is selected from dS004, dS005, dS006, dS007, dS008, dS009, dS010, dS011, dS012, dS013, dS014, dS015, dS016, dS017, dS018, dS019, dS020, dS021, dS022, dS023, dS024, dS025, dS026, dS027, dS028, dS029, dS030, dS031, dS032, dS033, dS034, dS035, dS036, dS037, dS038, dS039, dS040, dS041, dS042, dS043, dS044, dS045, dS046, dS047, dS048, dS049, dS050, dS051, dS052, dS053, dS054, dS055, dS056, dS057, dS058, dS059, dS060, dS061,

dS062, dS063, dS064, dS065, dS066, dS067, dS068, dS069, dS070, dS071, dS072, dS073, dS074, dS075, dS076, dS077, dS078, dS079, dS080, dS081, dS082, dS083, dS084, dS085, dS086, dS087, dS088, dS089, dS090, dS091, dS092, dS093, dS094, dS095, dS096, dS097, dS098, dS099, dS279, dS280, dS281, dS282, dS283, dS284, dS285, dS286, dS287, or dS288.

In a second aspect of the present invention, an siRNA conjugate is provided, which comprises the siRNA as described in the embodiment according to the first aspect of the present invention and a ligand group, where the ligand group is conjugated to the siRNA, the ligand group comprises 1, 2, 3 or 4 GalNAc groups, and the number of the ligand group is 1, 2, 3, 4, 5, 6 or 7.

In some embodiments according to the second aspect of the present invention, the number of the ligand group is 1.

In some embodiments according to the second aspect of the present invention, the ligand group comprises 3 GalNAc groups. In some embodiments according to the second aspect of the present invention, the ligand group comprises L96. In some embodiments according to the second aspect of the present invention, the ligand group comprises NAG37.

In some embodiments according to the second aspect of the present invention, the siRNA conjugate is selected from dS301, dS302, dS303, dS304, dS305, dS306, dS307, dS316, dS319 or dS324.

In a third aspect of the present invention, a pharmaceutical composition is provided, which comprises the siRNA as described in the embodiment according to the first aspect of the present invention or the siRNA conjugate as described in the embodiment according to the second aspect of the present invention and a pharmaceutically acceptable carrier.

In a fourth aspect of the present invention, use of the siRNA as described in the embodiment according to the first aspect of the present invention, the siRNA conjugate as described in the embodiment according to the second aspect of the present invention, or the pharmaceutical composition according to the third aspect of the present invention in the manufacture of a medicament for treating diseases related to the expression of DGAT2.

In some embodiments according to the fourth aspect of the present invention, the diseases related to the expression of DGAT2 are lipid metabolism diseases.

In some embodiments according to the fourth aspect of the present invention, the diseases related to the expression of DGAT2 are non-alcoholic fatty liver disease and non-alcoholic steatohepatitis.

In a fifth aspect of the present invention, a method for treating diseases and/or disorders related to the expression of DGAT2 is provided. The method comprises administering a therapeutically effective amount of the siRNA, the siRNA conjugate and/or the pharmaceutical composition to a subject in need thereof.

In some embodiments according to the fifth aspect of the present invention, the diseases related to the expression of DGAT2 are lipid metabolism diseases.

In some embodiments according to the fifth aspect of the present invention, the diseases related to the expression of DGAT2 are non-alcoholic fatty liver disease and non-alcoholic steatohepatitis.

In a sixth aspect of the present invention, a method for inhibiting the expression of the DGAT2 gene in a cell is provided, comprising contacting an effective amount of the siRNA, the siRNA conjugate and/or the pharmaceutical composition with the cell, to inhibit the expression of the DGAT2 gene in the cell.

As described above, in addition to administration thereof, the siRNA and/or the siRNA conjugates characterized herein can be administered in combination with other known agents that are effective in inhibiting the expression of DGAT2. In any circumstances, the physician can adjust the administration amount and timing of the siRNA and/or the siRNA conjugate based on the results observed using standard efficacy measurements known in the art or described herein.

The present invention provides the following sequences, which are written and read in the direction of 5' to 3':

**Table 1.1 Sequences involved in the present invention**

| SEQ ID No. | Nucleotide sequence (5' to 3') |
|---|---|
| 1 | CGAUGGGUCCAGAAGAAGUU |
| 2 | GGUUAUUUAAAAGAAAUUAU |
| 3 | UGCUGACCACCAGGAACUAU |
| 4 | GACCACCAGGAACUAUAUCU |
| 5 | AUGGGUGUCUGUGGGUUAUU |
| 6 | UGGGUGUCUGUGGGUUAUUU |
| 7 | GGGUGUCUGUGGGUUAUUUA |
| 8 | GUGUCUGUGGGUUAUUUAAA |

(continued)

| SEQ ID No. | Nucleotide sequence (5' to 3') |
|---|---|
| 9 | UGGGUUAUUUAAAAGAAAUU |
| 10 | GGGUUAUUUAAAAGAAAUUA |
| 11 | CUGACCACCAGGAACUAUAU |
| 12 | ACCACCAGGAACUAUAUCUU |
| 13 | CCACCAGGAACUAUAUCUUU |
| 14 | CAGGAACUAUAUCUUUGGAU |
| 15 | GGGAGUGGCAAUGCUAUCAU |
| 16 | CAUGGGUGUCUGUGGGUUAU |
| 17 | GGUGUCUGUGGGUUAUUUAA |
| 18 | UGUCUGUGGGUUAUUUAAAA |
| 19 | GUCUGUGGGUUAUUUAAAAG |
| 20 | CUGUGGGUUAUUUAAAAGAA |
| 21 | GACUACUUUCCCAUCCAGCU |
| 22 | CUGGUGAAGACACACAACCU |
| 23 | GUGAAGACACACAACCUGCU |
| 24 | CCUGCUGACCACCAGGAACU |
| 25 | GCUGACCACCAGGAACUAUA |
| 26 | GAAUGGGAGUGGCAAUGCUA |
| 27 | GAGUGGCAAUGCUAUCAUCA |
| 28 | AGUGGCAAUGCUAUCAUCAU |
| 29 | GGCAAUGCUAUCAUCAUCGU |
| 30 | AGCUCCAUGCCUGGCAAGAA |
| 31 | GUCCAGAAGAAGUUCCAGAA |
| 32 | UCAUGGGUGUCUGUGGGUUA |
| 33 | GUGGGUUAUUUAAAAGAAAU |
| 34 | AGUUAGAUGAUUCACUUUUU |
| 35 | CACUUGGCUGGUGUUUGACU |
| 36 | AUUUUGCUAAACCAUUACAA |
| 37 | UGCAGUGCCAUCCUCAUGUA |
| 38 | CAUGUCAGACUUUUGUAUAU |
| 39 | CCAGAAAUACAUUGGUUUCG |
| 40 | ACAGAAGUGAGCAAGAAGUU |
| 41 | CCGGGACACCAUAGACUAUU |
| 42 | GACACCAUAGACUAUUUGCU |
| 43 | GAAGUGUACAAGCAGGUGAU |
| 44 | UGCUAAACCAUUACAAUGUU |
| 45 | CACCAUAGACUAUUUGCUUU |
| 46 | AUUACAAUGUUAGGUCUUUU |
| 47 | CUUCUGAGCAGCAGAUUAGU |

(continued)

| SEQ ID No. | Nucleotide sequence (5' to 3') |
|---|---|
| 48 | GUGUACAAGCAGGUGAUCUU |
| 49 | CGCUACUUUCGAGACUACUU |
| 50 | CAUUACAAUGUUAGGUCUUU |
| 51 | GCCAUCCUCAUGUACAUAUU |
| 52 | CAUCCUCAUGUACAUAUUCU |
| 53 | GUACAUAUUCUGCACUGAUU |
| 54 | UACUUCACUUGGCUGGUGUU |
| 55 | ACUUCACUUGGCUGGUGUUU |
| 56 | GCUACUUUCGAGACUACUUU |
| 57 | CGGGACACCAUAGACUAUUU |
| 58 | CGGAACCGCAAGGGCUUUGU |
| 59 | CCAUUACAAUGUUAGGUCUU |
| 60 | CAAUGUUAGGUCUUUUUUAA |
| 61 | GUUCUAGGUGGUGGCUAAAU |
| 62 | UCUAGGUGGUGGCUAAAUCU |
| 63 | UGGCUAAAUCUGGGCCUAAU |
| 64 | CUGAAACUGCAGGACCAGUU |
| 65 | GUGGAGUAACUGGUUUUUCU |
| 66 | CCCAAGCCUCACUUUUCUGU |
| 67 | CAGUGCCAUCCUCAUGUACA |
| 68 | AGUGCCAUCCUCAUGUACAU |
| 69 | UCAUGUACAUAUUCUGCACU |
| 70 | UGCACUGAUUGCUGGCUCAU |
| 71 | CUCUACUUCACUUGGCUGGU |
| 72 | CUGGGUGCCUUCUGCAACUU |
| 73 | UGAGGGAGUACCUGAUGUCU |
| 74 | UACCUGAUGUCUGGAGGUAU |
| 75 | GCCGGGACACCAUAGACUAU |
| 76 | GGGACACCAUAGACUAUUUG |
| 77 | ACACCAUAGACUAUUUGCUU |
| 78 | GCUUUCAAAGAAUGGGAGUG |
| 79 | CAAAGAAUGGGAGUGGCAAU |
| 80 | GAAGAAGUUCCAGAAAUACA |
| 81 | UGGUUUCGCCCCAUGCAUCU |
| 82 | GGUUUCGCCCCAUGCAUCUU |
| 83 | GCUAAACCAUUACAAUGUUA |
| 84 | UACAAUGUUAGGUCUUUUUU |
| 85 | ACAAUGUUAGGUCUUUUUUA |
| 86 | UGUUAGGUCUUUUUUAAGAA |

(continued)

| SEQ ID No. | Nucleotide sequence (5' to 3') |
|---|---|
| 87 | CACUUCUCAUACAAGCCCCU |
| 88 | ACUGCAGGACCAGUUUCUCU |
| 89 | CUCUGCCAAGGGGAGGAGUU |
| 90 | ACCAUGAGCUAGGUGGAGUA |
| 91 | CCAUGAGCUAGGUGGAGUAA |
| 92 | GCUAGGUGGAGUAACUGGUU |
| 93 | GGUGGAGUAACUGGUUUUUC |
| 94 | GCAGCAGAUUAGUUCCAAAG |
| 95 | GAUUAGUUCCAAAGCAGGUG |
| 96 | CCGAACCCAAGCCUCACUUU |
| 201 | ACACCAUAGACUAUUUGCUUU |

**Table 1.2 Sequences involved in the present invention**

| SEQ ID No. | Nucleotide sequence (5' to 3') |
|---|---|
| 97 | AACUUCUUCUGGACCCAUCGGC |
| 98 | AUAAUUUCUUUUAAAUAACCCA |
| 99 | AUAGUUCCUGGUGGUCAGCAGG |
| 100 | AGAUAUAGUUCCUGGUGGUCAG |
| 101 | AAUAACCCACAGACACCCAUGA |
| 102 | AAAUAACCCACAGACACCCAUG |
| 103 | UAAAUAACCCACAGACACCCAU |
| 104 | UUUAAAUAACCCACAGACACCC |
| 105 | AAUUUCUUUUAAAUAACCCACA |
| 106 | UAAUUUCUUUUAAAUAACCCAC |
| 107 | AUAUAGUUCCUGGUGGUCAGCA |
| 108 | AAGAUAUAGUUCCUGGUGGUCA |
| 109 | AAAGAUAUAGUUCCUGGUGGUC |
| 110 | AUCCAAAGAUAUAGUUCCUGGU |
| 111 | AUGAUAGCAUUGCCACUCCCAU |
| 112 | AUAACCCACAGACACCCAUGAC |
| 113 | UUAAAUAACCCACAGACACCCA |
| 114 | UUUUAAAUAACCCACAGACACC |
| 115 | CUUUUAAAUAACCCACAGACAC |
| 116 | UUCUUUUAAAUAACCCACAGAC |
| 117 | AGCUGGAUGGGAAAGUAGUCUC |
| 118 | AGGUUGUGUGUCUUCACCAGCU |
| 119 | AGCAGGUUGUGUGUCUUCACCA |
| 120 | AGUUCCUGGUGGUCAGCAGGUU |
| 121 | UAUAGUUCCUGGUGGUCAGCAG |

(continued)

| SEQ ID No. | Nucleotide sequence (5' to 3') |
|---|---|
| 122 | UAGCAUUGCCACUCCCAUUCUU |
| 123 | UGAUGAUAGCAUUGCCACUCCC |
| 124 | AUGAUGAUAGCAUUGCCACUCC |
| 125 | ACGAUGAUGAUAGCAUUGCCAC |
| 126 | UUCUUGCCAGGCAUGGAGCUCA |
| 127 | UUCUGGAACUUCUUCUGGACCC |
| 128 | UAACCCACAGACACCCAUGACA |
| 129 | AUUUCUUUUAAAUAACCCACAG |
| 130 | AAAAAGUGAAUCAUCUAACUGG |
| 131 | AGUCAAACACCAGCCAAGUGAA |
| 132 | UUGUAAUGGUUUAGCAAAAUUG |
| 133 | UACAUGAGGAUGGCACUGCAGG |
| 134 | AUAUACAAAAGUCUGACAUGGU |
| 135 | CGAAACCAAUGUAUUUCUGGAA |
| 136 | AACUUCUUGCUCACUUCUGUGG |
| 137 | AAUAGUCUAUGGUGUCCCGGCU |
| 138 | AGCAAAUAGUCUAUGGUGUCCC |
| 139 | AUCACCUGCUUGUACACUUCAU |
| 140 | AACAUUGUAAUGGUUUAGCAAA |
| 141 | AAAGCAAAUAGUCUAUGGUGUC |
| 142 | AAAAGACCUAACAUUGUAAUGG |
| 143 | ACUAAUCUGCUGCUCAGAAGGC |
| 144 | AAGAUCACCUGCUUGUACACUU |
| 145 | AAGUAGUCUCGAAAGUAGCGCC |
| 146 | AAAGACCUAACAUUGUAAUGGU |
| 147 | AAUAUGUACAUGAGGAUGGCAC |
| 148 | AGAAUAUGUACAUGAGGAUGGC |
| 149 | AAUCAGUGCAGAAUAUGUACAU |
| 150 | AACACCAGCCAAGUGAAGUAGA |
| 151 | AAACACCAGCCAAGUGAAGUAG |
| 152 | AAAGUAGUCUCGAAAGUAGCGC |
| 153 | AAAUAGUCUAUGGUGUCCCGGC |
| 154 | ACAAAGCCCUUGCGGUUCCGCA |
| 155 | AAGACCUAACAUUGUAAUGGUU |
| 156 | UUAAAAAAGACCUAACAUUGUA |
| 157 | AUUUAGCCACCACCUAGAACAG |
| 158 | AGAUUUAGCCACCACCUAGAAC |
| 159 | AUUAGGCCCAGAUUUAGCCACC |
| 160 | AACUGGUCCUGCAGUUUCAGGA |

(continued)

| SEQ ID No. | Nucleotide sequence (5' to 3') |
|---|---|
| 161 | AGAAAAACCAGUUACUCCACCU |
| 162 | ACAGAAAAGUGAGGCUUGGGUU |
| 163 | UGUACAUGAGGAUGGCACUGCA |
| 164 | AUGUACAUGAGGAUGGCACUGC |
| 165 | AGUGCAGAAUAUGUACAUGAGG |
| 166 | AUGAGCCAGCAAUCAGUGCAGA |
| 167 | ACCAGCCAAGUGAAGUAGAGCA |
| 168 | AAGUUGCAGAAGGCACCCAGGC |
| 169 | AGACAUCAGGUACUCCCUCAAC |
| 170 | AUACCUCCAGACAUCAGGUACU |
| 171 | AUAGUCUAUGGUGUCCCGGCUG |
| 172 | CAAAUAGUCUAUGGUGUCCCGG |
| 173 | AAGCAAAUAGUCUAUGGUGUCC |
| 174 | CACUCCCAUUCUUUGAAAGCAA |
| 175 | AUUGCCACUCCCAUUCUUUGAA |
| 176 | UGUAUUUCUGGAACUUCUUCUG |
| 177 | AGAUGCAUGGGGCGAAACCAAU |
| 178 | AAGAUGCAUGGGGCGAAACCAA |
| 179 | UAACAUUGUAAUGGUUUAGCAA |
| 180 | AAAAAAGACCUAACAUUGUAAU |
| 181 | UAAAAAAGACCUAACAUUGUAA |
| 182 | UUCUUAAAAAAGACCUAACAUU |
| 183 | AGGGGCUUGUAUGAGAAGUGGC |
| 184 | AGAGAAACUGGUCCUGCAGUUU |
| 185 | AACUCCUCCCCUUGGCAGAGAA |
| 186 | UACUCCACCUAGCUCAUGGUGG |
| 187 | UUACUCCACCUAGCUCAUGGUG |
| 188 | AACCAGUUACUCCACCUAGCUC |
| 189 | GAAAAACCAGUUACUCCACCUA |
| 190 | CUUUGGAACUAAUCUGCUGCUC |
| 191 | CACCUGCUUUGGAACUAAUCUG |
| 192 | AAAGUGAGGCUUGGGUUCGGGG |
| 194 | AAGCAAAUAGUCUAUGGUGU |
| 195 | UAAGCAAAUAGUCUAUGGUGU |
| 196 | UUAAAUAACCCACAGACACC |
| 197 | UUUAAAUAACCCACAGACACC |
| 198 | UGUAUUUCUGGAACUUCUUC |
| 199 | UUGUAUUUCUGGAACUUCUUC |
| 200 | AAAGCAAAUAGUCUAUGGUGU |

The present invention provides siRNAs and siRNA conjugates set forth in a table below, and the following sequences are written and read in the direction of 5' to 3'. The abbreviations used in the table are as follows: (invAb) is an inverted abasic nucleotide, * indicates that two nucleotides are connected by a phosphorothioate linkage, lowercase letters (*e.g.,* a, u, g, and c) indicate that the nucleotides is modified with 2'-methoxy (*i.e.,* 2'-O-methyl), capital letters A, U, C, G and T followed by "f" indicate that the nucleotide is modified with 2'-fluoro, and VP is vinyl phosphonate. L96 represents a ligand group having a structure below:

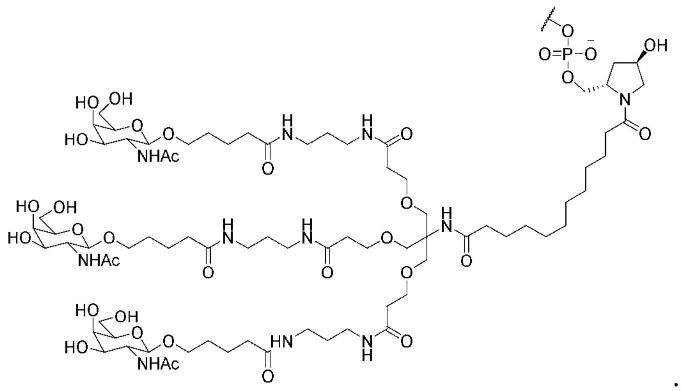

.

NAG37 represents a ligand group having a structure below:

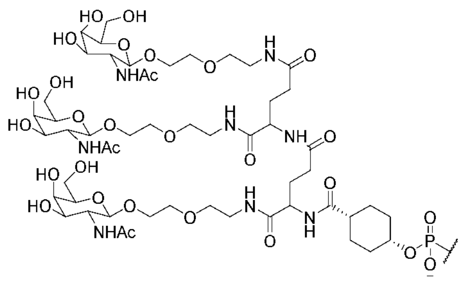

**Table 2. siRNA involved in the present invention**

| siRNA **No.** | **Sense strand (5'→3')** | **Anti-sense strand (5'→3')** |
|---|---|---|
| dS004 | (invAb)*guggguuaUfUfUfaaaagaaauu*(invAb) | a*Af*u*UfuCfuuuuaAfaUfaAfcCfcAf*c |
| dS005 | (invAb)*uguggguuAfUfUfuaaaagaaau*(invAb) | a*Uf*u*UfcUfuuuaaAfuAfaCfcCfaCf*a |
| dS006 | (invAb)*gguccagaAfGfAfaguuccagaa*(invAb) | u*Uf*c*UfgGfaacuuCfuUfcUfgGfaCf*c |
| dS007 | (invAb)*aguguacaAfGfCfaggugaucuu*(invAb) | a*Af*g*AfuCfaccugCfuUfgUfaCfaCf*u |
| dS008 | (invAb)*ggguuauuUfAfAfaagaaauuau*(invAb) | a*Uf*a* AfuUfucuuuUfaAfaUfaAfcCf*c |
| dS009 | (invAb)*ugaccaccAfGfGfaacuauaucu*(invAb) | a*Gf*a*UfaUfaguucCfuGfgUfgGfuCf*a |
| dS010 | (invAb)*ccgaugggUfCfCfagaagaaguu*(invAb) | a*Af*c*UfuCfuucugGfaCfcCfaUfcGf*g |
| dS011 | (invAb)*caugggugUfCfUfguggguuauu*(invAb) | a*Af*u*AfaCfccacaGfaCfaCfcCfaUf*g |
| dS012 | (invAb)*auggguguCfUfGfuggguuauuu*(invAb) | a*Af*a*UfaAfcccacAfgAfcAfcCfcAf*u |
| dS013 | (invAb)*uggguGucUfGfUfggguuauuua*(invAb) | u*Af*a*AfuAfacccaCfaGfaCfaCfcCf*a |
| dS014 | (invAb)*ggugucugUfGfGfguuauuuaaa*(invAb) | u*Uf*u*AfaAfuaaccCfaCfaGfaCfaCf*c |
| dS015 | (invAb)*uggguuauUfUfAfaaagaaauua*(invAb) | u*Af*a*UfuUfcuuuuAfaAfuAfaCfcCf*a |
| dS016 | (invAb)*cugcugacCfAfCfcaggaacuau*(invAb) | a*Uf*a*GfuUfccuggUfgGfuCfaGfcAf*g |
| dS017 | (invAb)*gcugaccaCfCfAfggaacuauau*(invAb) | a*Uf*a*UfaGfuuccuGfgUfgGfuCfaGf*c |
| dS018 | (invAb)*gaccaccaGfGfAfacuauaucuu*(invAb) | a* Af*g* AfuAfuaguuCfcUfgGfuGfgUf*c |

(continued)

| siRNA **No.** | **Sense strand (5'→3')** | **Anti-sense strand (5'→3')** |
|---|---|---|
| dS019 | (invAb)*ccaggaacUfAfUfaucuuuggau*(invAb) | a*Uf*c*CfaAfagauaUfaGfuUfcCfuGf*g |
| dS020 | (invAb)*ugggagugGfCfAfaugcuaucau*(invAb) | a*Uf*g*AfuAfgcauuGfcCfaCfuCfcCf*a |
| dS021 | (invAb)*cuacuccuUfUfGfgagagaauga*(invAb) | u*Cf*a*UfuCfucuccAfaAfgGfaGfuAf*g |
| dS022 | (invAb)*ucaugggUGfUfCfuguggguuau*(invAb) | a*Uf*a*AfcCfcacagAfcAfcCfcAfuGf*a |
| dS023 | (invAb)*gggugucuGfUfGfgguuauuuaa*(invAb) | u*Uf*a*AfaUfaacccAfcAfgAfcAfcCf*c |
| dS024 | (invAb)*gugucuguGfGfGfuuauuuaaaa*(invAb) | u*Uf*u*UfaAfauaacCfcAfcAfgAfcAf*c |
| dS025 | (invAb)*gucuguggGfUfUfauuuaaaaga*(invAb) | u*Cf*u*UfuUfaaauaAfcCfcAfcAfgAf*c |
| dS026 | (invAb)*cuuucgagAfCfUfacuuucccau*(invAb) | a*Uf*g*GfgAfaaguaGfuCfuCfgAfaAf*g |
| dS027 | (invAb)*accugcugAfCfCfaccaggaacu*(invAb) | a*Gf*u*UfcCfuggugGfuCfaGfcAfgGf*u |
| dS028 | (invAb)*ugcugaccAfCfCfaggaacuaua*(invAb) | u*Af*u*AfgUfuccugGfuGfgUfcAfgCf*a |
| dS029 | (invAb)*accaccagGfAfAfcuauaucuuu*(invAb) | a* Af*a*GfaUfauaguUfcCfuGfgUfgGf*u |
| dS030 | (invAb)*gaacuauaUfCfUfuuggauacca*(invAb) | u*Gf*g*UfaUfccaaaGfaUfaUfaGfuUf*c |
| dS031 | (invAb)*ucaaagaaUfGfGfgaguggcaau*(invAb) | a*Uf*u*GfcCfacuccCfaUfuCfuUfuGf*a |
| dS032 | (invAb)*agaaugggAfGfUfggcaaugcua*(invAb) | u*Af*g*CfaUfugccaCfuCfcCfaUfuCf*u |
| dS033 | (invAb)*gaaugggaGfUfGfgcaaugcuau*(invAb) | a*Uf*a*GfcAfuugccAfcUfcCfcAfuUf*c |
| dS034 | (invAb)*ggaguggcAfAfUfgcuaucauca*(invAb) | u*Gf*a*UfgAfuagcaUfuGfcCfaCfuCf*c |
| dS035 | (invAb)*gaguggcaAfUfGfcuaucaucau*(invAb) | a*Uf*g*AfuGfauagcAfuUfgCfcAfcUf*c |
| dS036 | (invAb)*gagcuccaUfGfCfcuggcaagaa*(invAb) | u*Uf*c*UfuGfccaggCfaUfgGfaGfcUf*c |
| dS037 | (invAb)*uacuccuuUfGfGfagagaaugaa*(invAb) | u*Uf*c*AfuUfcucucCfaAfaGfgAfgUf*a |
| dS038 | (invAb)*ucuguggGUfUfAfuuuaaaagaa*(invAb) | u*Uf*c*UfuUfuaaauAfaCfcCfaCfaGf*a |
| dS039 | (invAb)*ucacuuggCfUfGfguguuugacu*(invAb) | a*Gf*u*CfaAfacaccAfgCfcAfaGfuGf*a |
| dS040 | (invAb)*gaaacugcAfGfGfaccaguuucu*(invAb) | a*Gf*a*AfaCfuggucCfuGfcAfgUfuUf*c |
| dS041 | (invAb)*ccaugucaGfAfCfuuuuguauau*(invAb) | a*Uf*a*UfaCfaaaagUfcUfgAfcAfuGf*g |
| dS042 | (invAb)*acauauucUfGfCfacugauugcu*(invAb) | a*Gf*c* AfaUfcagugCfaGfaAfuAfuGf*u |
| dS043 | (invAb)*cacagaagUfGfAfgcaagaaguu*(invAb) | a*Af*c*UfuCfuugcuCfaCfuUfcUfgUf*g |
| dS044 | (invAb)*guuuuucuUfGfGfguggcugaug*(invAb) | c* Af*u*CfaGfccaccCfaAfgAfaAfaAf*c |
| dS045 | (invAb)*guucuguuAfUfCfucuugaugag*(invAb) | c*Uf*c*AfuCfaagagAfuAfaCfaGfaAf*c |
| dS046 | (invAb)*guuaucucUfUfGfaugagaucau*(invAb) | a*Uf*g*AfuCfucaucAfaGfaGfaUfaAf*c |
| dS047 | (invAb)*guggaguaAfCfUfgguuuuucuu*(invAb) | a* Af*g* AfaAfaaccaGfuUfaCfuCfcAf*c |
| dS048 | (invAb)*cuucugcaAfCfUfucagcacaga*(invAb) | u*Cf*u*GfuGfcugaaGfuUfgCfaGfaAf*g |
| dS049 | (invAb)*cuggccuuCfUfGfagcagcagau*(invAb) | a*Uf*c*UfgCfugcucAfgAfaGfgCfcAf*g |
| dS050 | (invAb)*gaagaaguUfCfCfagaaauacau*(invAb) | a*Uf*g*UfaUfuucugGfaAfcUfuCfuUf*c |
| dS051 | (invab)*ccacagaaGfUfGfagcaagaagu*(invAb) | a*Cf*u*UfcUfugcucAfcUfuCfuGfuGf*g |
| dS052 | (invAb)*ggacaccaUfAfGfacuauuugcu*(invAb) | a*Gf*c* AfaAfuagucUfaUfgGfuGfuCf*c |
| dS053 | (invAb)*ugaaguguAfCfAfagcaggugau*(invAb) | a*Uf*c*AfcCfugcuuGfuAfcAfcUfuCf*a |
| dS054 | (invAb)*cuucugagCfAfGfcagauuaguu*(invAb) | a*Af*c*UfaAfucugcUfgCfuCfaGfaAf*g |
| dS055 | (invAb)*cuucacuuGfGfCfugguguuuga*(invAb) | u*Cf*a*AfaCfaccagCfcAfaGfuGfaAf*g |
| dS056 | (invAb)*gccgggacAfCfCfauagacuauu*(invAb) | a*Af*u*AfgUfcuaugGfuGfuCfcCfgGf*c |
| dS057 | (invAb)*acaccauaGfAfCfuauuugcuuu*(invAb) | a*Af*a*GfcAfaauagUfcUfaUfgGfuGf*u |

(continued)

| siRNA **No.** | **Sense strand (5'→3')** | **Anti-sense strand (5'→3')** |
|---|---|---|
| dS058 | (invAb)*gcuuucaaAfGfAfaugggagugg*(invAb) | c*Cf*a*CfuCfccauuCfuUfuGfaAfaGf*c |
| dS059 | (invAb)*cauuacaaUfGfUfuaggucuuuu*(invAb) | a*Af*a*AfgAfccuaaCfaUfuGfuAfaUf*g |
| dS060 | (invAb)*ccuucugaGfCfAfgcagauuagu*(invAb) | a*Cf*u* AfaUfcugcuGfcUfcAfgAfaGf*g |
| dS061 | (invAb)*gcgcuacuUfUfCfgagacuacuu*(invAb) | a*Af*g*UfaGfucucgAfaAfgUfaGfcGf*c |
| dS062 | (invAb)*ccauuacaAfUfGfuuaggucuuu*(invAb) | a* Af*a*GfaCfcuaacAfuUfgUfaAfuGf*g |
| dS063 | (invAb)*auuacaauGfUfUfaggucuuuuu*(invAb) | a*Af*a*AfaGfaccuaAfcAfuUfgUfaAf*u |
| dS064 | (invAb)*cagugccaUfCfCfucauguacau*(invAb) | a*Uf*g*UfaCfaugagGfaUfgGfcAfcUf*g |
| dS065 | (invAb)*ugccauccUfCfAfuguacauauu*(invAb) | a*Af*u*AfuGfuacauGfaGfgAfuGfgCf*a |
| dS066 | (invAb)*ccauccucAfUfGfuacauauucu*(invAb) | a*Gf*a*AfuAfuguacAfuGfaGfgAfuGf*g |
| dS067 | (invAb)*uguacauaUfUfCfugcacugauu*(invAb) | a* Af*u*CfaGfugcagAfaUfaUfgUfaCf*a |
| dS068 | (invAb)*cuacuucaCfUfUfggcugguguu*(invAb) | a* Af*c* AfcCfagccaAfgUfgAfaGfuAf*g |
| dS069 | (invAb)*uacuucacUfUfGfgcugguguuu*(invAb) | a*Af*a*CfaCfcagccAfaGfuGfaAfgUf*a |
| dS070 | (invAb)*cgcuacuuUfCfGfagacuacuuu*(invAb) | a*Af*a*GfuAfgucucGfaAfaGfuAfgCf*g |
| dS071 | (invAb)*ccgggacaCfCfAfuagacuauuu*(invAb) | a* Af*a*UfaGfucuauGfgUfgUfcCfcGf*g |
| dS072 | (invAb)*gcggaaccGfCfAfagggcuuugu*(invAb) | a*Cf*a*AfaGfcccuuGfcGfgUfuCfcGf*c |
| dS073 | (invAb)*ugguuucgCfCfCfcaugcaucuu*(invAb) | a*Af*g*AfuGfcauggGfgCfgAfaAfcCf*a |
| dS074 | (invAb)*accauuacAfAfUfguuaggucuu*(invAb) | a*Af*g*AfcCfuaacaUfuGfuAfaUfgGf*u |
| dS075 | (invAb)*acaauguuAfGfGfucuuuuuuaa*(invAb) | u*Uf*a*AfaAfaagacCfuAfaCfaUfuGf*u |
| dS076 | (invAb)*uaagaaggAfAfAfaagucaguau*(invAb) | a*Uf*a*CfuGfacuuuUfuCfcUfuCfuUf*a |
| dS077 | (invAb)*uguucuagGfUfGfguggcuaaau*(invAb) | a*Uf*u*UfaGfccaccAfcCfuAfgAfaCf*a |
| dS078 | (invAb)*guggcuaaAfUfCfugggccuaau*(invAb) | a*Uf*u*AfgGfcccagAfuUfuAfgCfcAf*c |
| dS079 | (invAb)*accaugagCfUfAfgguggaguaa*(invAb) | u*Uf*a*CfuCfcaccuAfgCfuCfaUfgGf*u |
| dS080 | (invAb)*caugagcuAfGfGfuggaguaacu*(invAb) | a*Gf*u*UfaCfuccacCfuAfgCfuCfaUf*g |
| dS081 | (invAb)*gguggaguAfAfCfugguuuuucu*(invAb) | a*Gf*a*AfaAfaccagUfuAfcUfcCfaCf*c |
| dS082 | (invAb)*cucauguaCfAfUfauucugcacu*(invAb) | a*Gf*u*GfcAfgaauaUfgUfaCfaUfgAf*g |
| dS083 | (invAb)*cauguacaUfAfUfucugcacuga*(invAb) | u*Cf*a*GfuGfcagaaUfaUfgUfaCfaUf*g |
| dS084 | (invAb)*cugguguuUfGfAfcuggaacaca*(invAb) | u*Gf*u*GfuUfccaguCfaAfaCfaCfcAf*g |
| dS085 | (invAb)*ccugggugCfCfUfucugcaacuu*(invAb) | a*Af*g*UfuGfcagaaGfgCfaCfcCfaGf*g |
| dS086 | (invAb)*guaccugaUfGfUfcuggagguau*(invAb) | a*Uf*a*CfcUfccagaCfaUfcAfgGfuAf*c |
| dS087 | (invAb)*agccgggaCfAfCfcauagacuau*(invAb) | a*Uf*a*GfuCfuauggUfgUfcCfcGfgCf*u |
| dS088 | (invAb)*gacaccauAfGfAfcuauuugcuu*(invAb) | a*Af*g*CfaAfauaguCfuAfuGfgUfgUf*c |
| dS089 | (invAb)*gaaugaagUfGfUfacaagcaggu*(invAb) | a*Cf*c*UfgCfuuguaCfaCfuUfcAfuUf*c |
| dS090 | (invAb)*caauuuugCfUfAfaaccauuaca*(invAb) | u*Gf*u*AfaUfgguuuAfgCfaAfaAfuUf*g |
| dS091 | (invAb)*auguuaggUfCfUfuuuuuaagaa*(invAb) | u*Uf*c*UfuAfaaaaaGfaCfcUfaAfcAf*u |
| dS092 | (invab)*cacuucucAfUfAfcaagccccuu*(invab) | a*Af*g*GfgGfcuuguAfuGfaGfaAfgUf*g |
| dS093 | (invAb)*ccugaaacUfGfCfaggaccaguu*(invAb) | a*Af*c*UfgGfuccugCfaGfuUfuCfaGf*g |
| dS094 | (invAb)*ucucugccAfAfGfgggaggaguu*(invAb) | a*Af*c*UfcCfuccccUfuGfgCfaGfaGf*a |
| dS095 | (invAb)*gagcuaggUfGfGfaguaacuggu*(invAb) | a*Cf*c*AfgUfuacucCfaCfcUfaGfcUf*c |
| dS096 | (invAb)*cugagcagCfAfGfauuaguucca*(invAb) | u*Gf*g*AfaCfuaaucUfgCfuGfcUfcAf*g |

(continued)

| siRNA **No.** | **Sense strand (5'→3')** | **Anti-sense strand (5'→3')** |
|---|---|---|
| dS097 | (invAb)*cagauuagUfUfCfcaaagcaggu*(invAb) | a*Cf*c*UfgCfuuuggAfaCfuAfaUfcUf*g |
| dS098 | (invAb)*cccgaaccCfAfAfgccucacuuu*(invAb) | a*Af*a*GfuGfaggcuUfgGfgUfuCfgGf*g |
| dS099 | (invAb)*gaacccaaGfCfCfucacuuuucu*(invAb) | a*Gf*a*AfaAfgugagGfcUfuGfgGfuUf*c |
| dS279 | (invAb)*uggguuauUfUfAfaaagaaauua*(invAb) | u*Af*a*UfuUfcuuuuAfaAfuAfaCfcCf*a |
| dS280 | (invAb)*uguggguuAfUfUfuaaaagaaau*(invAb) | a*Uf*u*UfcUfuuuaaAfuAfaCfcCfaCf*a |
| dS281 | (invAb)*guggguuaUfUfUfaaaagaaauu*(invAb) | a*Af*u*UfuCfuuuuaAfaUfaAfcCfcAf*c |
| dS282 | (invAb)*caugggugUfCfUfgugguuauu*(invAb) | a*Af*u*AfaCfccacaGfaCfaCfcCfaUf*g |
| dS283 | (invAb)*augggguguCfUfGfugguuauuu*(invAb) | a*Af*a*UfaAfcccacAfgAfcAfcCfcAf*u |
| dS284 | (invAb)*gugucuguGfGfGfuuauuuaaaa*(invAb) | u*Uf*u*UfaAfauaacCfcAfcAfgAfcAf*c |
| dS285 | (invAb)*ugggugucUfGfUfggguuauuua*(invAb) | u*Af*a*AfuAfacccaCfaGfaCfaCfcCf*a |
| dS286 | (invAb)*ggugucugUfGfGfguuauuuaaa*(invAb) | u*Uf*u*AfaAfuaaccCfaCfaGfaCfaCf*c |
| dS287 | (invAb)*gcugaccaCfCfAfggaacuauau*(invAb) | a*Uf*a*UfaGfuuccuGfgUfgGfuCfaGf*c |
| dS288 | (invAb)*ggguuauuUfAfAfaagaaauuau*(invAb) | a*Uf*a* AfuUfucuuuUfaAfaUfaAfcCf*c |
| dS301 | (invAb)*gaagaaguUfCfCfagaaauacau*(invAb)[L96] | a*Uf*g*UfaUfuucugGfaAfcUfuCfuUf*c |
| dS302 | (invAb)*acaccauaGfAfCfuauuugcuuu*(invAb)[L96] | a* Af*a*GfcAfaauagUfcUfaUfgGfuGf*u |
| dS303 | (invAb)*uguacauaUfUfCfugcacugauu*(invAb)[L96] | a* Af*u*CfaGfugcagAfaUfaUfgUfaCf*a |
| dS304 | (invAb)*cgcuacuuUfCfGfagacuacuuu*(invAb)[L96] | a*Af*a*GfuAfgucucGfaAfaGfuAfgCf*g |
| dS305 | [NAG37](invAb)*uggguuauUfUfAfaaagaaauua*(invAb ) | u*Af*a*UfuUfcuuuuAfaAfuAfaCfcCf*a |
| dS306 | [NAG37](invAb)*uguggguuAfUfUfuaaaagaaau*(invAb ) | a*Uf*u*UfcUfuuuaaAfuAfaCfcCfaCf*a |
| dS307 | (invAb)*ggugucugUfGfGfguuauuuaaa*(invAb)*[L96] | Vpu*Uf*Uf*AfaauaaccCfaCfaGfacac*c |
| dS316 | (invAb)*gaagaaguUfCfCfagaaauacaa[L96] | Vpu*Uf*g*UfaUfuucugGfaAfcUfuCfuUf* c |
| dS319 | (invAb)*acaccauaGfAfCfuauuugcuua[L96] | Vpu*Af*a*GfcAfaauagUfcUfaUfgGfuGf* u |
| dS324 | (invAb)*ggugucugUfGfGfguuauuuaaa[L96] | Vpu*Uf*Uf*AfaauaaccCfaCfaGfacac*c |

**Beneficial effects**

The siRNA, the siRNA conjugate and the pharmaceutical composition provided in the present invention have a high stability, a high DGAT2 mRNA inhibitory activity, and a low off-target effect, and/or can significantly treat diseases and/or disorders related to the expression of DGAT2.

The siRNA, the siRNA conjugate and the pharmaceutical composition provided in the present invention show excellent target gene inhibitory activity in in-vitro experiments. In some embodiments, the siRNA, the siRNA conjugate and the pharmaceutical composition provided in the present invention show, at a concentration of 100 nM, a target gene inhibitory activity of ≥80%, 85%, 90%, or 95% in in-vitro experiments. In some embodiments, the siRNA or the siRNA conjugate provided in the present invention shows, at a concentration of 10 nM, a target gene inhibitory activity of ≥50%, 60%, 70%, 80%, 90%, 91%, or 92% on the target gene in in-vitro experiments. In some embodiments, the siRNA or the siRNA conjugate provided in the present invention shows, at a concentration of 0.5 nM, a target gene inhibitory activity of ≥40%, 50%, 60%, 70%, 80%, or 90% on the target gene in in-vitro experiments. In some embodiments, the siRNA or the siRNA conjugate provided in the present invention shows a target gene inhibitory activity characterized by an $IC_{50}$ value of ≤20 pM, 15 pM, 14 pM, 13 pM, 12 pM, 11 pM, 10 pM, 9 pM, 8 pM, 7 pM, 6 pM, 5 pM, 4 pM, 3 pM or 2.5 pM in in-vitro experiments. In some embodiments, the siRNA or the siRNA conjugate provided in the present invention shows a target gene inhibitory activity in in-vitro experiments, which is characterized by the reduced expression level of the mRNA of the target gene measured by qPCR. In some embodiments, the siRNA or the siRNA conjugate provided in the present invention shows a target gene inhibitory activity in in-vitro experiments, which is measured in human liver cancer cells (such as Huh7 cells). In some embodiments, the siRNA or the siRNA conjugate provided in the present invention shows a target gene inhibitory activity in in-vitro experiments, which is measured in human hepatocytes (such as primary human hepatocytes (PHHs)). In some embodiments, the siRNA or the siRNA conjugate provided in the present invention shows a

target gene inhibitory activity in in-vitro experiments, which is measured by the method in Example 2 or 3. In some embodiments, the target gene is DGAT2.

The siRNA, the siRNA conjugate and the pharmaceutical composition provided in the present invention show excellent target gene inhibitory activity in in-vivo experiments. In some embodiments, the target gene is DGAT2.

In some embodiments, the siRNA, the siRNA conjugate and the pharmaceutical composition provided in the present invention show, after 7 days of administration, an average inhibition rate of ≥10%, 20%, 30%, 40%, 50%, 60%, 70% or 80% on the target gene in in-vivo experiments. In some embodiments, the siRNA, the siRNA conjugate and the pharmaceutical composition provided in the present invention show, after 28 days of administration, an average inhibition rate of ≥10%, 20%, 30%, 40%, 50%, 60% or 65% on the target gene in in-vivo experiments. In some embodiments, the siRNA, the siRNA conjugate and the pharmaceutical composition provided in the present invention show a target gene inhibitory activity in in-vivo experiments, which is characterized by the reduced expression level of the mRNA of the target gene in the liver of wild-type mice measured by qPCR. In some embodiments, the siRNA, the siRNA conjugate and the pharmaceutical composition provided in the present invention show a target gene inhibitory activity in in-vivo experiments, which is measured by the method in Example 4 or 6.

In some embodiments, the siRNA, the siRNA conjugate and the pharmaceutical composition provided in the present invention show, after 15 days of administration, an average inhibition rate of ≥50%, 60%, 70%, 80%, 90%, 95% or 97% on the target gene in in-vivo experiments. In some embodiments, the siRNA, the siRNA conjugate and the pharmaceutical composition provided in the present invention show a target gene inhibitory activity in in-vivo experiments, which is characterized by the reduced expression level of the mRNA of the target gene in the liver of a mice model receiving hydrodynamic injection of a human target gene carrying plasmid via the tail vein measured by qPCR. In some embodiments, the siRNA, the siRNA conjugate and the pharmaceutical composition provided in the present invention show a target gene inhibitory activity in in-vivo experiments, which is measured by the method in Example 5, 7 or 8.

In some embodiments, the siRNA, the siRNA conjugate and the pharmaceutical composition provided in the present invention show improvement on non-alcoholic steatohepatitis of a western diet in human target gene carrying mice in in-vivo experiments. In some embodiments, the siRNA, the siRNA conjugate and the pharmaceutical composition provided in the present invention show improvement on non-alcoholic steatohepatitis of a western diet in human DGAT2 carrying mice in in-vivo experiments, which is measured by the method in Example 9.

In some embodiments, the siRNA, the siRNA conjugate and the pharmaceutical composition provided in the present invention show, after 29 days of administration, an average inhibition rate of ≥50%, 60%, 70%, 80%, 90% or 95% on the target gene in in-vivo experiments. In some embodiments, the siRNA, the siRNA conjugate and the pharmaceutical composition provided in the present invention show, after 57 days of administration, an average inhibition rate of ≥60%, 70%, 80%, 90% or 95% on the target gene in in-vivo experiments. In some embodiments, the siRNA, the siRNA conjugate and the pharmaceutical composition provided in the present invention show a target gene inhibitory activity in in-vivo experiments, which is characterized by the reduced expression level of the mRNA of the target gene in the liver of non-naive Cynomolgus macaque measured by qPCR. In some embodiments, the siRNA, the siRNA conjugate and the pharmaceutical composition provided in the present invention show a target gene inhibitory activity in in-vivo experiments, which is measured by the method in Example 10 or 11. The siRNA, the siRNA conjugate and the pharmaceutical composition provided in the present invention show no obvious off-target effect. The off-target effect can be, for example, inhibition of normal gene expression of non-target genes. It is considered that if the binding/inhibition of off-target gene expression is lower than 50%, 40%, 30%, 20% or 10% compared with the effect on the target gene, the off-target effect is not significant.

## Definitions

Unless otherwise specified, the following terms and phrases used in the present invention are intended to have the following meanings. A particular phrase or term should not be considered uncertain or unclear unless specifically defined, but should be understood in its ordinary meaning. When a trade name appears herein, it is intended to refer to the corresponding product or active ingredient.

In the present invention, "optionally" or "optional" means that the event or condition described later may or may not occur, and the description includes the situation in which the event or condition occurs and the situation in which the event or condition does not occur. For example, "optionally substituted" "alkyl" includes "alkyl" and "substituted alkyl" as defined below. It will be understood by those skilled in the art that for any group containing one or more substituents, these groups are not intended to introduce any substitution or substitution pattern that is spatially impractical, synthetically infeasible and/or inherently unstable.

In the present invention, the "subject" refers to any animal, such as a mammal or a marsupial. The subject in the present invention includes, but is not limited to, human, a non-human primate (e.g., rhesus monkey or other types of macaques), mice, pigs, horses, cattle, rats or any kind of domestic fowls.

In the present invention, the "treatment" refers to a method to obtain beneficial or expected results, including, but

not limited to, therapeutic benefits. The "therapeutic benefit" means to eradicate or improve the potential disorders treated. In addition, the therapeutic benefits are obtained by eradicating or improving one or more physiological symptoms related to the potential disorder, so that improvement is observed in the subject, although the subject may still suffer from the potential disorder.

In the present invention, the siRNA or the siRNA conjugate can be used to treat diseases. In some embodiments of the present invention, the diseases are diseases related to the expression of DGAT2. In some embodiments of the present invention, the diseases related to the expression of DGAT2 are lipid metabolism disease. In some embodiments of the present invention, the diseases related to the expression of DGAT2 are non-alcoholic fatty liver disease and non-alcoholic steatohepatitis.

In the present invention, the "small interfering RNA", "siRNA", "RNAi" or "iRNA agent" can be used interchangeably, and refers to a double-stranded ribonucleic acid molecule that is long enough to trigger interferon response and form a RNA-induced silencing complex (RISC) and short enough that it does not induce harmful interferon response in human cells (for example, the siRNA agent or its cleavage product can down-regulate a target gene by, for example, inducing RNAi of the target RNA, where the target may include endogenous or pathogenic target RNA). In some embodiments of the present invention, the siRNA is at least partially complementary to the coding sequence in the target gene expressed in the cell. In some embodiments of the present invention, after the siRNA is delivered to a cell expressing the gene, the siRNA can inhibit or block the expression of the gene in vitro or in vivo. Usually the siRNA contains a duplex region with less than 60, 50, 40 or 30 complementary base pairs; and preferably, a duplex region with 10, 11, 12, 13, 14, 15, 16, 17, 18, 19, 20, 21 or 22 complementary base pairs. In some embodiments of the present invention, the sense strand and anti-sense strand of the siRNA are each independently 15-30 nucleotides, and the formed complementary duplex region has a length of 10, 11, 12, 13, 14, 15, 16, 17, 18, 19, 20, 21 or 22 base pairs. In some embodiments of the present invention, the sense strand and the anti-sense strand of the siRNA are completely complementarily paired and has a length of 15-30 base pairs. In some embodiments of the present invention, the sense strand and the anti-sense strand of the siRNA are completely complementarily paired and has a length of 17, 18, 19, 20, 21 or 22 base pairs.

In the present invention, the siRNA "targeting" the target gene (or a designated fragment of the target gene) means that the anti-sense strand of the siRNA is at least partially complementary to at least a part of RNA transcribed by the target gene (or a designated fragment thereof). In some embodiments of the present invention, the siRNA provided in the present invention targets the diacylglycerol acyltransferase 2 (Acyl CoA:Diacylglycerol Acyltransferase 2, DGAT2) gene. In some embodiments of the present invention, the anti-sense strand of the siRNA provided in the present invention is at least partially complementary to the targeted fragment of RNA transcribed from the DGAT2 gene. In some embodiments of the present invention, the anti-sense strand of the siRNA provided in the present invention is completely complementary to the targeted fragment of RNA transcribed from the DGAT2 gene.

The targeted fragment position of the DGAT2 gene targeted by the anti-sense strand of the siRNA provided in the present invention may be based on the position in the DGAT2 gene transcript. In some embodiments of the present invention, the accession number of the DGAT2 gene transcript in NCBI database is NM_032564.5. In some embodiments of the present invention, the DGAT2 gene transcript has a sequence as set forth in SEQ ID NO: 193. In some embodiments of the present invention, the siRNA provided in the present invention targets a sequence of any 17 to 22 (for example, 20 or 21) consecutive nucleotides at positions 450 to 600 in SEQ ID NO: 193. In some embodiments of the present invention, the siRNA provided in the present invention targets a sequence of any 17 to 22 (for example, 20 or 21) consecutive nucleotides at positions 475-512 or 529-562 in SEQ ID NO: 193. In some embodiments of the present invention, the siRNA provided in the present invention targets positions 489-509 in SEQ ID NO: 193.

In some embodiments of the present invention, the siRNA provided in the present invention targets a sequence of any 17 to 22 (for example, 20 or 21) consecutive nucleotides at positions 600-700 in SEQ ID NO: 193. In some embodiments of the present invention, the siRNA provided in the present invention targets a sequence of any 17 to 22 (for example, 20 or 21) consecutive nucleotides at positions 614-639 or 660-696 in SEQ ID NO: 193.

In some embodiments of the present invention, the siRNA provided in the present invention targets a sequence of any 17 to 22 (for example, 20 or 21) consecutive nucleotides at positions 700-800 in SEQ ID NO: 193. In some embodiments of the present invention, the siRNA provided in the present invention targets a sequence of any 17 to 22 (for example, 20 or 21) consecutive nucleotides at positions 715-768 in SEQ ID NO: 193.

In some embodiments of the present invention, the siRNA provided in the present invention targets a sequence of any 17 to 22 (for example, 20 or 21) consecutive nucleotides at positions 800-1200 in SEQ ID NO: 193. In some embodiments of the present invention, the siRNA provided in the present invention targets a sequence of any 17 to 22 (for example, 20 or 21) consecutive nucleotides at positions 832-1002, 1042-1083, or 1102-1155 in SEQ ID NO: 193. In some embodiments of the present invention, the siRNA provided in the present invention targets a sequence of any 17 to 22 (for example, 20 or 21) consecutive nucleotides at positions 870-889, 870-890, 1114-1133, or 1114-1134 in SEQ ID NO: 193. In some embodiments of the present invention, the siRNA provided in the present invention targets positions 870-889, 870-890, 1114-1133 or 1114-1134 in SEQ ID NO: 193.

In some embodiments of the present invention, the siRNA provided in the present invention targets a sequence of

any 17 to 22 (for example, 20 or 21) consecutive nucleotides at positions 1400-1650 in SEQ ID NO: 193. In some embodiments of the present invention, the siRNA provided in the present invention targets a sequence of any 17 to 22 (for example, 20 or 21) consecutive nucleotides at positions 1450-1466, 1450-1488, 1491-1547, or 1576-1607 in SEQ ID NO: 193. In some embodiments of the present invention, the siRNA provided in the present invention targets a sequence of any 17-22 (for example, 20 or 21) consecutive nucleotides at positions 1450-1470, 1451-1471, 1452-1472, 1453-1473, 1454-1474, 1455-1475, 1456-1476, 1457-1477, 1458-1478, 1459-1479, 1460-1480, 1461-1481, 1462-1482, 1463-1483, 1464-1484, 1465-1485, 1466-1486, 1467-1487, 1468-1488, 1469-1489 or 1470-1490 in SEQ ID NO: 193. In some embodiments of the present invention, the siRNA provided in the present invention targets positions 1450-1470, 1451-1471, 1452-1472, 1453-1473, 1454-1474, 1455-1475, 1456-1476, 1457-1477, 1458-1478, 1459-1478, 1459-1479, 1460-1480, 1461-1481, 1462-1482, 1463-1483, 1464-1484, 1465-1485, 1466-1486, 1467-1487, 1468-1488, 1469-1489 or 1470-1490 in SEQ ID NO: 193.

In some embodiments of the present invention, the siRNA provided in the present invention targets a sequence of any 17 to 22 (for example, 20 or 21) consecutive nucleotides at positions 1700-1900 in SEQ ID NO: 193. In some embodiments of the present invention, the siRNA provided in the present invention targets a sequence of any 17 to 22 (for example, 20 or 21) consecutive nucleotides at positions 1750-1840 in SEQ ID NO: 193.

In some embodiments of the present invention, the siRNA provided in the present invention targets a sequence of any 17 to 22 (for example, 20 or 21) consecutive nucleotides at positions 2000-2100 in SEQ ID NO: 193. In some embodiments of the present invention, the siRNA provided in the present invention targets a sequence of any 17 to 22 (for example, 20 or 21) consecutive nucleotides at positions 2045-2089 in SEQ ID NO: 193.

In some embodiments of the present invention, the siRNA provided in the present invention targets a sequence of any 17 to 22 (for example, 20 or 21) consecutive nucleotides at positions 2200-2400 in SEQ ID NO: 193. In some embodiments of the present invention, the siRNA provided in the present invention targets a sequence of any 17 to 22 (for example, 20 or 21) consecutive nucleotides at positions 2209-2273 or 2326-2376 in SEQ ID NO: 193. In the present invention, "inhibition" means that when a given gene is expressed, the gene expression is reduced when a cell, cell group or tissue is treated with the siRNA, the siRNA conjugate and the pharmaceutical composition of the present invention, compared with the cell, cell group or tissue that has not received such a treatment. In the present invention, the "inhibition", "reduction", "silencing", "down-regulation", "suppression" and other similar terms can be used interchangeably and include any level of inhibition. Preferably, the inhibition includes statistically significant inhibition or clinically significant inhibition.

In the present invention, "conjugation" means that two or more chemical moieties with respectively specific functions are covalently connected to each other. Accordingly, the "conjugate" refers to a compound formed by covalent connection of the various chemical moieties. Further, the "siRNA conjugate" refers to a compound formed by covalent connection of one or more chemical moieties with specific functions (such as a ligand group) to a siRNA. Herein, the siRNA conjugate of the present invention is sometimes referred to simply as "conjugate", which should be understood as the full name of the siRNA conjugate according to the context. In some embodiments of the present invention, the ligand group can be connected to the phosphate group (including the covalent connection of the ligand group to the atom at position 3’ or 5’ of the nucleotide by a phosphodiester linkage), the hydroxyl group at position 2’, and the hydroxyl group at position 5’, or the base of any nucleotide in the siRNA. In some embodiments of the present invention, the ligand group can also be connected to the hydroxyl group at the 3’ position, where the nucleotides are connected by a 2’-5’ phosphodiester linkage.

In the present invention, the "ligand group" includes a pharmaceutically acceptable scaffold group and at least one targeting group, and the siRNA, the scaffold group and the targeting group are connected in sequence, that is, in the form of "siRNA-scaffold group-targeting group". In some embodiments, there are 2-4 targeting groups. The siRNA molecule may be covalently or non-covalently conjugated to the ligand group, for example, it may be covalently conjugated to the ligand group. The conjugation site of the siRNA to the ligand group may be located at the 3’ end or 5’ end of the sense strand of the siRNA, or in an internal sequence of the siRNA. In some embodiments, the conjugation site of the siRNA to the ligand group is located at the 3’ end or the 5’ end of the sense strand of the siRNA. In some embodiments, the conjugation site of the siRNA to the ligand group is located at the 5’ end of the sense strand of the siRNA. In some embodiments, the conjugation site of the siRNA to the ligand group is located at the 3’ end of the sense strand of the siRNA.

In the present invention, the "targeting group" refers to a targeting agent for cells or tissues of animals or human bodies, for example, lectins, glycoproteins, lipids or proteins (such as antibodies) that bind to particular cell types such as kidney cells. The targeting group can be thyroid stimulating hormone, melanocyte stimulating hormone, lectins, glycoproteins, surfactant protein A, mucin carbohydrate, polyvalent lactose, polyvalent galactose, N-acetyl-galactosamine (i.e. GalNAc), N-acetyl-glucosamine, polyvalent mannose, polyvalent fucose, glycosylated polyamino acid, polyvalent galactose, transferrin, bisphosphonate, polyglutamic acid, polyaspartic acid, lipids, cholesterol, steroids, bile acid, folic acid, vitamin B12, biotins, RGD peptide, RGD peptide mimetics or aptamers.

In the present invention, the "scaffold group" refers to a structure connecting two parts of a compound, which usually includes a direct bond or an atom such as oxygen or sulfur, for example, -NR-, -C(=O)-, -C(=O)NH-, -S(=O)-, $-SO_2-$, $-SO_2NH-$, or an atomic chain, for example, substituted or unsubstituted alkyl, substituted or unsubstituted alkenyl,

substituted or unsubstituted alkynyl, arylalkyl, arylalkenyl, arylalkynyl, heteroarylalkyl, heteroarylalkenyl, heteroarylalkynyl, heterocycloalkyl, heterocycloalkenyl, heterocycloalkynyl, aryl, heteroaryl, heterocyclyl, cycloalkyl, cycloalkenyl, alkylarylalkyl, alkylarylalkenyl, alkylarylalkynyl, alkenylarylalkyl, alkenylarylalkenyl, alkenylarylalkynyl, alkynylarylalkyl, alkynylarylalkenyl, alkynylarylalkynyl, alkylheteroarylalkyl, alkylheteroarylalkenyl, alkylheteroarylalkynyl, alkenylheteroarylalkyl, alkenylheteroarylalkenyl, alkenylheteroarylalkynyl, alkynylheteroarylalkyl, alkynylheteroarylalkenyl, alkynylheteroarylalkynyl, alkylheterocycloalkyl, alkylheterocycloalkenyl, alkylheterocycloalkynyl, alkenylheterocycloalkyl, alkenylheterocycloalkenyl, alkenylheterocycloalkynyl, alkynylheterocycloalkyl, alkynylheterocycloalkenyl, alkynylheterocycloalkynyl, alkylaryl, alkenylaryl, alkynylaryl, alkylheteroaryl, alkenylheteroaryl, and alkynylheteroaryl, in which one or more methylene can be interrupted or terminated by -O-, -S-, -S(=O)-, $-SO_2-$, $-N(R)_2-$, -C(=O)-, a cleavable linking group, substituted or unsubstituted aryl, substituted or unsubstituted heteroaryl, or substituted or unsubstituted heterocyclyl, in which R is hydrogen, acyl, an aliphatic or substituted aliphatic group. The scaffold group may be a divalent, a trivalent, a tetravalent, a pentavalent, a hexavalent or a higher group. The scaffold group contains 0, 1, 2 or 3 branch points. In some embodiments of the present invention, the branch point is C, CH, $-CH_2-$, N, - NH-, -O-, -S-, or -C(=O)-. In some embodiments of the present invention, the scaffold group is tetravalent, and the scaffold group has 1 branch point. Further, the scaffold group is

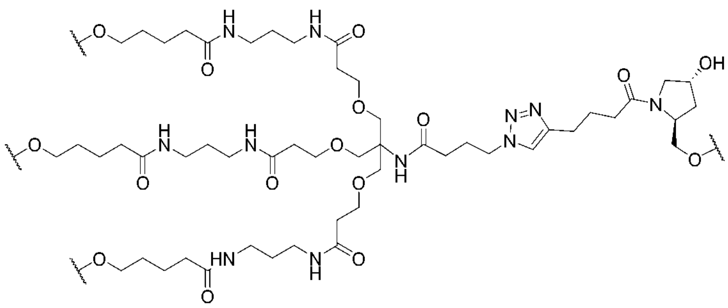

.

In some embodiments of the present invention, the scaffold group is divalent, and the scaffold group has 0 branch point. Further, the scaffold group is

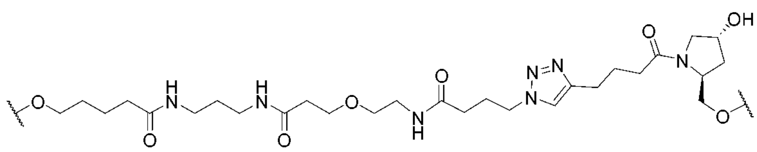

.

In some embodiments of the present invention, the ligand group is [NAG37], and the siRNA conjugate has a structure shown below:

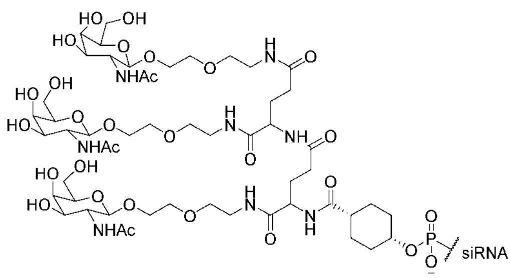

.

In some embodiments of the present invention, the ligand group is [L96], and the siRNA conjugate has a structure shown below:

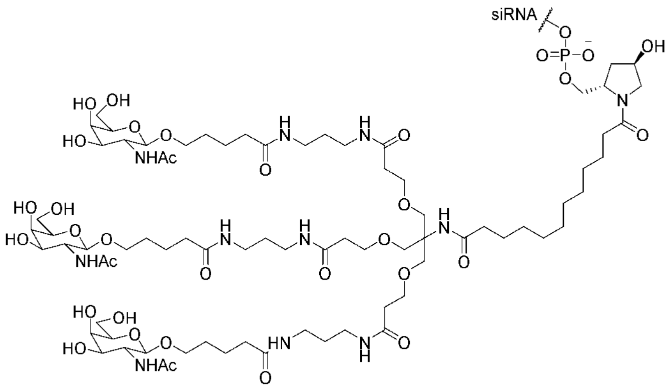

.

In the present invention, "complementary" or "reverse complementary" can be used interchangeably, and refers to the structural relationship between two nucleotides (for example, on two opposite nucleic acid strands or on the opposite regions of a single nucleic acid strand), which allows the two nucleotides to form a base pair with each other (for example, a purine nucleotide of a nucleic acid complementary to a pyrimidine nucleotide of an opposite nucleic acid can form a base pair by hydrogen bonding with each other). In some embodiments of the present invention, complementary nucleotides can be base-paired by Watson-Crick base pairing or in any other manner that allows the formation of a stable duplex. In some embodiments of the present invention, two nucleic acid strands may form multiple complementary duplex regions. In some embodiments of the present invention, in DNA, adenine (A) is always paired with thymine (T), and in RNA, adenine (A) is always with uracil (U); and guanine (G) is always paired with cytosine (C).

In the present invention, "mispairing" means that in a double-stranded nucleic acid molecule, the bases at corresponding positions are not paired in a complementary manner.

In the present invention, all strands/sequences are written and read in the direction of 5' to 3'.

In the present invention, the siRNA may include one or more modified nucleotides. In some embodiments, the sense strand of the siRNA may include one or more modified nucleotides. In the present invention, the sense strand of the siRNA may include 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9, 1 to 8, 1 to 7, 1 to 6, 1 to 5, 1 to 4, 1 to 3 or 1 to 2 modified nucleotides. In some embodiments, the sense strand of the siRNA may include 21, 20, 19, 18, 17, 16, 15, 14, 13, 12, 11, 10, 9, 8, 7, 6, 5, 4, 3, 2 or 1 modified nucleotide. In some embodiments, the sense strand of the siRNA may include 20 to 21, 19 to 21, 18 to 21, 17 to 21, 16 to 21, 15 to 21, 14 to 21, 13 to 21, 12 to 21, 11 to 21, 10 to 21, 9 to 21, 8 to 21, 7 to 21, 6 to 21, 5 to 21, 4 to 21, 3 to 21, 2 to 21 or 1 to 21 modified nucleotides.

In some embodiments, the anti-sense strand of the siRNA may include one or more modified nucleotides. In the present invention, the anti-sense strand of the siRNA may include 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9, 1 to 8, 1 to 7, 1 to 6, 1 to 5, 1 to 4, 1 to 3 or 1 to 2 modified nucleotides. In some embodiments, the anti-sense strand of the siRNA may include 21, 20, 19, 18, 17, 16, 15, 14, 13, 12, 11, 10, 9, 8, 7, 6, 5, 4, 3, 2 or 1 modified nucleotide. In some embodiments, the anti-sense strand of the siRNA may include 20 to 21, 19 to 21, 18 to 21, 17 to 21, 16 to 21, 15 to 21, 14 to 21, 13 to 21, 12 to 21, 11 to 21, 10 to 21, 9 to 21, 8 to 21, 7 to 21, 6 to 21, 5 to 21, 4 to 21, 3 to 21, 2 to 21 or 1 to 21 modified nucleotides.

In some embodiments, the sense strand of the siRNA includes 21 modified nucleotides, and the anti-sense strand includes 21 modified nucleotides. In some embodiments, the sense strand of the siRNA includes 18 modified nucleotides, and the anti-sense strand includes 13 modified nucleotides. In some embodiments, the sense strand of the siRNA includes 18 modified nucleotides, and the anti-sense strand includes 15 modified nucleotides. In some embodiments, the sense strand of the siRNA includes 21 modified nucleotides, the anti-sense strand includes 21 modified nucleotides, and the sense strand and the anti-sense strand include no natural nucleotides.

In the present invention, "G", "C", "A", "T" and "U" refer to nucleotides containing guanine, cytosine, adenine, thymine and uracil as bases. In the present invention, A, U, C, G and T preceded by "d" represent 2'- deoxyribonucleic acids, and represent ribonucleic acids when they are not preceded by "d". For example, dA is deoxyadenine, dU is deoxyuracil, dC is deoxycytosine, dG is deoxyguanine, and dT is deoxythymine. In the present invention, A, U, C, G and T followed by "f" indicate that the nucleotide is modified with 2'- fluoro. For example, Af is 2'-fluoroadenine, Uf is 2'-fluorouracil, Cf is 2'-fluorocytosine, Gf is 2'- fluoroguanine, and Tf is 2'- fluorothymine. Lowercase letters (for example, a, u, g, and c) indicate that the nucleotide is modified with 2'-methoxy (that is, 2'-O-methyl). For example, a is 2'- methoxyadenine, u is 2'- methoxyuracil, c is 2'-methoxycytosine, and g is 2'- methoxyguanine. In the present invention, the mark * between A, U, C, G, and T indicates that two nucleotides are connected by a phosphorothioate linkage. For example, "A*dU g A Uf G" means that starting from the 5' end of the sequence A-U-G-A-U-G, the nucleotides at positions 1 and 2 are connected by a phosphothiodiester linkage, the nucleotide at position 2 is a deoxyribonucleic acid, the nucleotide at position 3 is modified with 2'-methoxy, and the nucleotide at position 5 is modified with 2'-fluoro.

In some embodiments, the sense strand of the siRNA provided in the present invention has 21 nucleotides, in which 18 nucleotides are nucleotides modified with 2'-methoxy, and 3 nucleotides are nucleotides modified with 2'-fluoro; and the anti-sense strand has 21 nucleotides, in which 13 nucleotides are nucleotides modified with 2'-methoxy, and 8 nucleotides are nucleotides modified with 2'-fluoro. In some embodiments, the sense strand of the siRNA provided in the present invention has 21 nucleotides, and starting from the 5' end of the sense strand, the nucleotides at positions 1 to 8 and positions 12 to 21 are nucleotides modified with 2'-methoxy, and the nucleotides at positions 9 to 11 are nucleotides modified with 2'-fluoro; and/or the anti-sense strand of the siRNA provided in the present invention has 21 nucleotides, and starting from the 5' end of the anti-sense strand, the nucleotides at positions 1, 3, 5, 7 to 11, 13, 15, 17, 19 and 21 are nucleotides modified with 2'-methoxy, and the nucleotides at positions 2, 4, 6, 12, 14, 16, 18 and 20 are nucleotides modified with 2'-fluoro.

In the present invention, the siRNA may include an abasic nucleotide. As used herein, the term "abasic" refers to a moiety lacking a base or having other chemical groups to replace the base at position 1'. In some embodiments, the abasic nucleotide may be an inverted abasic nucleotide. In some embodiments, the sense strand of the siRNA includes an abasic nucleotide (for example, inverted abasic nucleotide) at the 3' end and/or 5' end. In some embodiments, the sense strand of the siRNA includes an inverted abasic nucleotide at both the 3' end and the 5' end. In some embodiments, the sense strand of the siRNA includes an inverted abasic nucleotide at the 5' end.

In the present invention, the siRNA may include one or more modified internucleoside linkages (including the connection between the siRNA and the ligand group, and the connection with the 3' terminal and/or 5' terminal abasic nucleotide). In some embodiments, the modified internucleoside linkage includes a modified phosphate group, such as phosphorothioate, phosphoselenate, boranophosphate, H-phosphonate, phosphoramidate, alkyl or aryl phosphonate, or phosphorodithioate. In some embodiments, the siRNA may include one or more phosphorothioate-modified internucleoside linkages (including the connection between the siRNA and the ligand group, and the connection with the 3' terminal and/or 5' terminal abasic nucleotide).

In some embodiments, the sense strand of the siRNA may include one or more phosphorothioate-modified internucleoside linkages (including the connection between the siRNA and the ligand group, and the connection with the 3' terminal and/or 5' terminal abasic nucleotide). In some embodiments, the sense strand of the siRNA includes a linkage with the inverted abasic nucleotide that is phosphorothioate at the 3' end and the 5' end, and other internucleoside linkages (including the connection between the siRNA and the ligand group) that are phosphodiester. In some embodiments, the sense strand of the siRNA includes a linkage with the inverted abasic nucleotide at the 3' end and the 5' end and a linkage between the inverted abasic nucleotide at the 3' end and the ligand group that are both phosphorothioate and other internucleoside linkages that are phosphodiester. In some embodiments, the sense strand of the siRNA includes a linkage with the inverted abasic nucleotide at the 5' end that is phosphorothioate, and other internucleoside linkages (including the connection between the siRNA and the ligand group) that are phosphodiester.

In some embodiments, the anti-sense strand of the siRNA includes a phosphorothioate-modified internucleoside linkage. In some embodiments, starting from the 5' end of the anti-sense strand of the siRNA, the connection between the nucleosides at positions 1 and 2, the nucleosides at positions 2 and 3, the nucleosides at positions 3 and 4, and the nucleosides at positions 20 and 21 are phosphorothioate, and other internucleoside linkages are phosphodiester.

In the present invention, the siRNA may include a 5'-terminal phosphate or phosphate analog modification ; and the 5'-terminal phosphate modification includes those that are compatible with RISC-mediated gene silencing. Suitable modifications include: 5'-monophosphate ($(HO)_2(O)P$-O-5'); 5'-diphosphate ($(HO)_2(O)P$-O-P(HO)(O)-O-5'); 5'-triphosphate ($(HO)_2(O)P$-O-(HO)(O)P-O-P(HO)(O)-O-5'); 5'-guanosine cap (7-methylated or non-methylated) (7m-G-O-5'-(HO)(O)P-O-(HO)(O)P-O-P(HO)(O)-O-5'); 5'-adenosine cap (Appp), and any modified or unmodified nucleotide cap structure (NO-5'-(HO)(O)P-O-(HO)(O)P-O-P(HO)(O)-O-5'); 5'-monophosphorothioate ($(HO)_2(S)P$-O-5'); 5'-monophosphorodithioate ( (HO)(HS)(S)P-O-5'), 5'-phosphorothioate ($(HO)_2(O)P$-S-5'); any additional combination of oxygen/sulfur substituted monophosphate, diphosphate and triphosphate (e.g., 5'-$\alpha$-thiotriphosphate, and 5'-$\gamma$-thiotriphosphate), 5'-phosphoramidate ($(HO)_2(O)P$-NH-5', $(HO)(NH_2)(O)P$-O-5'), 5'-alkylphosphonate (R=alkyl=methyl, ethyl, isopropyl, or propyl, for example, RP(OH)(O)-O-5'-, and $(OH)_2(O)P$-5'-$CH_2$-), 5'-vinyl phosphonate, and 5'-alkylether phosphonate (R=alkyl ether=methoxymethyl ($MeOCH_2^-$) or ethoxymethyl, for example, RP(OH)(O)-O-5'-). In some embodiments, the anti-sense strand of the siRNA includes a 5'-terminal vinyl phosphonate (VP) modification at the 5' end.

In the present invention, the "pharmaceutically acceptable carrier" may include, but is not limited to, an excipient and/or other components. The "excipient" is a pharmaceutically acceptable solvent, suspending agent or any other pharmaceutically inert medium for delivering one or more nucleic acids to an animal. Such reagents are well known in the art. The composition of the present invention may additionally contain other auxiliary components conventionally present in pharmaceutical compositions at a level of usage established in the art. Therefore, for example, the composition may contain an additional, compatible pharmaceutically active substance, such as an antipruritic agent, an astringent, a local anesthetic or an anti-inflammatory agent, or may contain additional substances that can be used to physically prepare various dosage forms of the composition of the present invention, such as a preservative, an antioxidant and a stabilizer. However, when added, such substances should not unduly interfere with the biological activities of the components in the

composition of the present invention. The preparation can be sterilized, and if necessary, it can be mixed with an auxiliary agent that will not adversely interact with the nucleic acid in the preparation, such as a preservative, a stabilizer, a wetting agent, an emulsifier, or a salt or a buffer that affects the osmotic pressure, and the like.

In the present invention, the siRNA or siRNA conjugate can be incorporated into a delivery vector, such as a liposome or a particle. Generally, as described herein, the siRNA composition is prepared in such a manner that is compatible with the intended method of administration. For example, in a specific embodiment, the composition is prepared by at least one of the following methods: spray drying, freeze drying, drying under vacuum, evaporation, fluidized bed drying or a combination of these technologies; or ultrasonic treatment, freeze-drying, condensation and other self-assembly with a lipid.

In the present invention, the term "liposome" refers to a vesicle formed of an amphiphilic lipid and arranged in at least one bilayer (for example, one bilayer or multiple bilayers). The liposome includes monolayer and multilayer vesicles with a membrane formed by a lipophilic material and an aqueous interior. The aqueous interior accommodates the siRNA or siRNA conjugate. The lipophilic material separates the aqueous interior from an aqueous exterior. The aqueous exterior typically does not contain the siRNA or siRNA conjugate, but in some examples, it may contain them. The liposome is suitable for transferring and delivering an active ingredient to a site of action. Because the liposome membrane is similar in structure to the biomembrane, when the liposome is applied to a tissue, the bilayer of the liposome is fused with the bilayer of the cell membrane. With the fusion of liposomes with cells, the internal aqueous content including the siRNA or siRNA conjugate is delivered to the cells, where the siRNA or siRNA conjugate can specifically bind to a target RNA and can mediate RNAi. In some cases, these liposomes are also specifically targeted to, for example, direct the siRNA or siRNA conjugate to a specific cell type.

An siRNA preparation can be formulated in combination with another agent (for example, another therapeutic agent or an agent for stabilizing the siRNA (e.g., a protein complexed with the siRNA to form an iRNP)). Other agents include a chelating agent (for example, EDTA (for example, to remove divalent cations, such as $Mg^{2+}$)), a salt, an RNase inhibitor (e.g., a broadly specific RNase inhibitor, such as RNAsin), and the like.

In some embodiments, the siRNA preparation includes another siNA compound, such as a second siRNA that can mediate RNAi against a second gene or against the same gene. Other preparations may include at least 3, 5, 10, 20, 50 or 100 or more different types of siRNAs. Such siRNAs can mediate RNAi against a similar number of different genes.

In some embodiments, the siRNA preparation includes at least one second therapeutic agent (e.g., an agent other than an RNA or a DNA). In some embodiments, the second therapeutic agent includes a drug for treating diseases such as non-alcoholic fatty liver disease, non-alcoholic steatohepatitis, obesity, insulin resistance, or other metabolic syndrome.

The iRNA agent of the present invention can be formulated for pharmaceutical use. The pharmaceutically acceptable composition includes a therapeutically effective amount of one or more of the dsRNA agents in any of the above embodiments, used alone or formulated with one or more pharmaceutically acceptable carriers (additives), excipients and/or diluents.

These pharmaceutical compositions can be specially prepared for administration in a solid or liquid form, including a form suitable for: (1) oral administration, such as oral gavage (aqueous or non-aqueous solution or suspension), tablets (such as tablets targeted for buccal, sublingual and systemic absorption), bolus, powders, granules, and pastes for application to the tongue; (2) parenteral administration, such as subcutaneous, intramuscular, intravenous or epidural injection, such as, for example, a sterile solution or suspension or a sustained-release preparation; (3) local application, for example, in the form of a cream, ointment or a controlled release patch or spray applied to the skin; (4) intravaginal or rectal administration, for example, in the form of a pessary, cream or foam; (5) sublingual; (6) intraocular administration; (7) percutaneous administration; or (8) transnasal administration. Delivery through subcutaneous or intravenous route can be particularly advantageous.

A composition including iRNA can be delivered to a subject through various routes. Exemplary routes include intravenous, subcutaneous, local, rectal, anal, vaginal, nasal, pulmonary, and ocular.

Depending on whether local or systemic treatment is desired and depending on the area to be treated, the composition of the present invention can be administered in various ways. The administration can be local (including ocular, vaginal, rectal, intranasal, and transdermal), oral or parenteral. Parenteral administration includes subcutaneous injection, intravenous infusion, intravenous injection, intraperitoneal or intramuscular injection, or intrathecal or intraventricular administration.

The route and site of administration can be selected to enhance the targeting. For example, to target muscle cells, intramuscular injection into the muscle of interest will be a logical choice. Lung cells can be targeted by administering iRNA as an aerosol. Vascular endothelial cells can be targeted by coating a balloon catheter with iRNA and mechanically introducing DNA.

The abbreviations used in the present invention are as follows: (invAb) is an inverted abasic nucleotide, and VP is vinyl phosphonate.

BRIEF DESCRIPTION OF ACCOMPANYING DRAWINGS

FIG. 1 shows the improvement on non-alcoholic steatohepatitis (NASH) induced by hDGAT2 combined with western diet by NAFLD activity score x dS319 over 4 weeks.
FIG. 2 shows the improvement on fibrosis in non-alcoholic steatohepatitis induced by hDGAT2 combined with western diet by NAFLD activity score x dS319 over 4 weeks.

DETAILED DESCRIPTION

The following examples are intended to illustrate the present invention, for better understanding of the present disclosure, instead of limiting the scope of the present invention. The modifications or transformations made to the elements of the present invention without departing from the spirit and essence of the present invention fall within the scope of the present invention. Unless otherwise specified, the reagents, kits and biological materials used in the present invention are all commercially available. Unless otherwise specified, the kits are used according to the instructions for use of the kits in the present invention.

**Example 1: Synthesis of siRNA molecule**

Oligoribonucleotides were synthesized according to phosphoramidite solid-state synthesis technology, on general-purpose controllable porous glass CPG. All 2'-modified RNA phosphoramidites and auxiliary reagents were commercially available. All phosphoramidites were dissolved in anhydrous acetonitrile and a molecular sieve was added. 5-ethylthio-1H-tetrazole (ETT) was used as an activator and the coupling was continued for 1.0 min. A 50 mM solution of 3-((dimethylamino-methylene) amino)-3H-1, 2, 4-dithiazole-3-thione (DDTT) in anhydrous acetonitrile/pyridine (v/v = 1/1) was used to generate a phosphorothioate bond, and the reaction time was 1.8 min. All the sequences were synthesized after the DMT group was finally removed.

Cleavage and deprotection of oligomer bound to CPG: After the termination of solid-state synthesis, the protective group was removed by treatment with an acetonitrile solution containing 20% diethylamine for 30 min without cleavage of the oligonucleotide from CPG. Then, the dried CPG was treated with concentrated aqueous ammonia at 40 degrees Celsius for 18 hrs. After centrifugation, the supernatant was transferred to a new tube and the CPG was washed with aqueous ammonia. The combined solution was concentrated to obtain a solid mixture.

Purification of single-stranded oligonucleotide: The oligomer was purified by HPLC and then anion-exchanged by using NanoQ. Buffer A was 10 mM sodium perchlorate solution, 20 mM Tris, 1 mM EDTA, pH7.4 and containing 20% acetonitrile, and buffer B was 500 mM sodium perchlorate, 20 mM Tris, 1 mM EDTA, pH 7.4 and containing 20% acetonitrile. The target product was separated and desalted by reversed-phase C18 column.

Annealing of single-stranded oligonucleotide to produce siRNA: The single-stranded oligonucleotide to be annealed was prepared into 200 μM with sterile RNase Free $H_2O$ (without RNA hydrolase). The annealing reaction system was set up as follows: A mixed solution with a total volume of 100 μL and a content of 10 nmol was incubated in a water bath at 95°C for 10 min (a demand of ≥ 100 nmol requires high temperature for 20 min)→ transferred to a water bath at 60°C and naturally cool down→the solution after annealing cannot be stored at high temperature. A complementary strand was formed by combining an equimolar single-stranded oligoribonucleotide solution.

**Example 2: In-vitro inhibitory activity test of test compounds on target gene DGAT2**

1. Materials and methods

1.1 Materials

1.1.1 Test compounds

The structures of the test compounds were shown in Table 2. The test compound was prepared into a 20 μM mother liquor with PBS.

1.1.2 Cell line

Huh7 cells. Huh7 cells were cultured in DMEM medium (Gibco article number 11965-092) containing 10% fetal bovine serum (FBS, ExCell Bio article number FSP500), 1% glutamine (GlutaMAX, Gibco article number 35050061), 1%

NEAA (gibco article number 11140050), and 1% penicillin-streptomycin (HyClone article number SV30010).

1.1.3 Main instruments

The main instruments used in this experiment include a fluorescence qPCR machine (Quanstudio 7 flex), a centrifuge (Beckman Allegra-X15R Centrifuge), and a cell counter (Countstar Rigel2).

1.1.4 Main reagents and materials

The main reagents used in this experiment include Lipofectamine™iRNAiMAX transfection reagent (INVITROGEN, article number 13778150), FastStart Universal Probe Mast (Roche, article number 04914058001), RNA extraction kit (Qiagen, article number 74182), FastKing cDNA first-strand synthesis kit (TianGen, article number KR116-02), and a 96-well plate (Costar 3599). TaqMan Gene Expression Assay, GAPDH (Thermo, article number Hs02786624_g1), DGAT2 (Thermo, article number Hs01045913_m1).

2. Experimental method

2.1 Compound transfection and plating

Huh7 cells ($2 \times 10^4$ cells/well) were inoculated into a 96-well plate, and siRNA was transfected into the cells while plating. 2 concentration points (10 nM, and 0.5 nM) were set for siRNA test, the cells were cultured overnight in an incubator at 37 °C with 5% $CO_2$, and 2 wells were measured in parallel. At the same time, a compound-free control group containing RNAiMAX was set up.

2.2 RNA extraction and reverse transcription

24 hrs after transfection, the medium was removed and the cells were collected for RNA extraction. Total RNA was extracted by using RNeasy® 96 Kit (QIAGEN-74182) according to the instructions for use of the kit. cDNA was synthesized by using FastKing RT Kit (with gDNase) (Tiangen-KR116-02) according to the instructions.

2.3 Detection of target gene mRNA expression level by qPCR

The target cDNA was detected by qPCR, and GAPDH cDNA as an internal control was detected in parallel. 8 μL of prepared PCR reaction solution and 2 μL of sample cDNA were added into a 384 well plate. The qPCR reaction procedure: heating at 95 °C for 10 min, and 40 cycles of heating at 95 °C for 15 sec and then at 60 °C for 1 min.

3. Data analysis

The expression level of the target gene mRNA in each sample was calculated by ΔΔCt relative quantitative method. The relative expression level of the target gene was expressed by $2^{-\Delta\Delta CT}$.

The calculation formula is as follows:

ΔCT = average Ct value of target gene - average Ct value of internal reference gene

$$\Delta\Delta CT = \Delta CT \text{ (treatment group) } - \Delta CT \text{ (RNAiMAX control group)}$$

$$\text{Relative expression level of target gene DGAT2} = 2^{-\Delta\Delta CT}$$

DGAT2 inhibition rate %= (1 - value determined in the treatment group/average in the RNAiMAX control group)* 100

GraphPad Prism software was used for plotting and analysis, and the inhibition rate was expressed as mean ± SD.

Test results:

**Table 3.1 In-vitro inhibitory activity test of test compounds on target gene DGAT2**

| Test compound No. | 10 nM | | 0.5 nM | |
|---|---|---|---|---|
| | Average inhibition rate (%) | Standard deviation | Average inhibition rate (%) | Standard deviation |
| dS004 | 93.27 | 1.25 | 82.37 | 3.14 |
| dS005 | 87.62 | 1.89 | 76.15 | 1.19 |
| dS006 | 85.39 | 1.27 | 42.4 | 5.95 |
| dS007 | 84.81 | 1.14 | 69.08 | 1.14 |
| dS008 | 91.54 | 2.24 | 74.38 | 1.65 |
| dS009 | 82.58 | 1.71 | 48.52 | 3.42 |
| dS010 | 75.77 | 0.5 | 50.51 | 0.76 |
| dS011 | 91.44 | 1.13 | 82.94 | 0.44 |
| dS012 | 93.81 | 0.09 | 79.21 | 0.01 |
| dS013 | 88.52 | 3.74 | 70.47 | 8.16 |
| dS014 | 92.58 | 0.15 | 71.32 | 2.73 |
| dS015 | 93.17 | 0.56 | 81.76 | 3.33 |
| dS017 | 90.11 | 1.04 | 77.86 | 1.26 |
| dS018 | 88.83 | 1.05 | 56.11 | 0.63 |
| dS021 | 79.27 | 3.05 | 54.49 | 4.66 |
| dS022 | 87.62 | 1.92 | 66.86 | 0.16 |
| dS023 | 87.59 | 1.43 | 74.25 | 0.53 |
| dS024 | 88.46 | 0.59 | 78.79 | 2.17 |
| dS025 | 94.39 | 1.01 | 82.93 | 0.54 |
| dS026 | 83.12 | 0.46 | 52.61 | 0.09 |
| dS028 | 84.07 | 3.56 | 60.98 | 1.86 |
| dS029 | 89.85 | 0.33 | 67.72 | 0.47 |
| dS030 | 89.54 | 1.41 | 54.11 | 3.35 |
| dS032 | 82.02 | 2.21 | 57.09 | 1.7 |
| dS033 | 82.77 | 1.61 | 53.07 | 10.89 |
| dS035 | 87.13 | 4.78 | 69.75 | 3.09 |
| dS038 | 88.65 | 2.83 | 77.85 | 2.1 |
| dS039 | 86.03 | 0.55 | 52.85 | 2.94 |
| dS050 | 93.59 | 0.39 | 78.39 | 0.36 |
| dS051 | 78.12 | 7.15 | 52.77 | 4.41 |
| dS052 | 83.9 | 1.74 | 53.26 | 1.2 |
| dS055 | 87.96 | 0.16 | 57.22 | 0.45 |
| dS057 | 89.3 | 2.73 | 83.89 | 2.05 |
| dS061 | 83.67 | 0.54 | 55.86 | 4.04 |
| dS064 | 84.46 | 2.67 | 65.97 | 6.42 |

(continued)

| Test compound No. | 10 nM | | 0.5 nM | |
|---|---|---|---|---|
| | Average inhibition rate (%) | Standard deviation | Average inhibition rate (%) | Standard deviation |
| dS065 | 84.03 | 1.52 | 62.36 | 2.46 |
| dS067 | 89.16 | 0.67 | 78.98 | 2.41 |
| dS069 | 88.39 | 3.09 | 63.73 | 13.5 |
| dS070 | 89.33 | 5.17 | 81.22 | 5.44 |
| dS087 | 78.54 | 0.64 | 52.13 | 4.45 |
| dS088 | 82.78 | 0.69 | 56.77 | 4.93 |

**Table 3.2 In-vitro inhibitory activity test of test compounds on target gene DGAT2**

| Test compound No. | Average inhibition rate (%) | | | | | | | | $IC_{50}$ (pM) |
|---|---|---|---|---|---|---|---|---|---|
| | 10000 pM | 3333 pM | 1111 pM | 370 pM | 123 pM | 41 pM | 14 pM | 4.6 pM | |
| dS011 | 95.1 | 92.3 | 87.5 | 85.1 | 72.4 | 59.5 | 32.8 | 21.9 | 2.9 |
| dS025 | 94.2 | 90.1 | 80.8 | 72.2 | 51.5 | 38.9 | 17.5 | 14.5 | 10.0 |
| dS004 | 95.3 | 91.5 | 90.4 | 86.8 | 72.8 | 63.5 | 29.8 | 27.5 | 2.9 |
| dS015 | 94.7 | 91.4 | 87.6 | 83.6 | 69.7 | 62.7 | 42.8 | 30.3 | 2.0 |
| dS012 | 96.1 | 93.4 | 86.5 | 81.5 | 67.0 | 54.0 | 28.3 | 23.9 | 4.1 |
| dS024 | 94.4 | 91.3 | 86.9 | 81.5 | 68.8 | 47.2 | 31.1 | 11.0 | 4.1 |
| dS017 | 89.1 | 87.5 | 83.8 | 77.9 | 64.2 | 38.3 | 16.9 | -2.8 | 6.2 |
| dS005 | 92.1 | 89.4 | 86.5 | 83.9 | 76.9 | 60.0 | 40.9 | 21.1 | 2.2 |
| dS008 | 94.7 | 91.5 | 86.5 | 77.0 | 61.2 | 37.1 | 15.3 | -6.5 | 7.0 |
| dS023 | 91.6 | 87.1 | 74.9 | 60.6 | 40.8 | 24.0 | 6.4 | -2.9 | 19.4 |
| dS014 | 91.1 | 88.0 | 83.3 | 73.9 | 65.5 | 41.8 | 23.0 | 5.8 | 5.8 |
| dS013 | 93.5 | 90.7 | 85.6 | 76.0 | 65.1 | 47.9 | 29.7 | 15.9 | 4.7 |
| dS035 | 88.3 | 86.5 | 81.4 | 68.3 | 51.6 | 36.4 | 11.9 | 13.1 | 11.0 |
| dS007 | 89.6 | 86.7 | 79.3 | 63.3 | 51.4 | 25.7 | 4.6 | 11.3 | 14.6 |
| dS029 | 88.6 | 86.3 | 73.1 | 60.4 | 45.2 | 25.8 | 10.9 | 6.2 | 18.4 |
| dS022 | 85.6 | 83.1 | 78.2 | 70.6 | 51.8 | 28.3 | 11.3 | 8.5 | 11.4 |
| dS028 | 81.4 | 73.1 | 57.2 | 41.9 | 21.4 | 7.1 | -8.9 | 0.1 | 60.9 |

**Table 3.3 In-vitro inhibitory activity test of test compounds on target gene DGAT2**

| Test compound No. | IC50 (pM) |
|---|---|
| dS319 | 16.26 |

**Conclusions:**

The double-stranded ribonucleic acid compound provided in the present application can significantly inhibit the level of DGAT2 mRNA in cells.

**Example 3: Inhibition rate of test compounds on target gene DGAT2 in primary human hepatocytes (PHHs)**

**Test compound:**

The test compound was prepared into a 100 μM/200 μM mother liquor with PBS.

**Cells:**

Human hepatocytes (PHHs). PHHs were revived and cultured in InvitroGRO CP medium (BIOIVT article number S0331) containing 10% fetal bovine serum (FBS, Gibco article number 10091148) and 1% penicillin-streptomycin (PS, HyClone article number SV30010).

**Main reagents and materials:**

The main reagents used in this experiment include FastStart Universal Probe Master (ROCHE article number 04914058001), RNA extraction kit (Qiagen article number 74182), HiScript III RT SuperMix for qPCR (+gDNA wiper) (Vazyme article number R323-01), 96-well plate (Costar article number 3599), GAPDH TagMan® Gene Expression Assays (60×) (Thermo article number Hs02786624_g1), and DGAT2 TagMan® Gene Expression Assays (60×) (Thermo article number Hs01045913_m1).

**Experimental method:**

Free uptake of compound and plating

PHHs ($5.4 \times 10^4$ cells/well) were inoculated into a 96-well plate, and siRNA was added into the cell while plating. The compound entered the cells through free uptake. 9 concentration points (100 nM, 20 nM, 4 nM, 0.8 nM, 0.16 nM, 0.032 nM, and 0.0064 nM) were set for siRNA test, and 2 wells were measured in parallel. At the same time, a compound-free control group containing PBS was set up. The cells were cultured in an incubator at 37°C with 5% $CO_2$.

RNA extraction and reverse transcription

48 hrs later, the medium was removed and the cells were collected for RNA extraction. Total RNA was extracted by using RNeasy® 96 Kit (QIAGEN-74182) according to the instructions for use of the kit. cDNA was synthesized by using HiScript III RT SuperMix for qPCR (+gDNA wiper) (Vazyme article number R323-01) following the instructions.

Detection of target gene mRNA expression level by qPCR

The target gen cDNA was detected by qPCR, and the internal reference gene GAPDH cDNA was also detected. 8 μL of prepared PCR reaction solution and 2 μL of sample cDNA were added into a 384 well plate. The qPCR reaction procedure: heating at 50°C for 2 min, heating at 95°C for 10 min, and 40 cycles of heating at 95°C for 15 sec and then at 60°C for 1 min.

**Experimental results:**

See Tables 4.1 and 4.2.

**Table 4.1 Inhibition rate of test compounds on target gene DGAT2 mRNA in primary human hepatocytes**

| Test compound No. | 100 nM | 10 nM | 1 nM |
|---|---|---|---|
| | **Average inhibition rate (%)** | **Average inhibition rate (%)** | **Average inhibition rate (%)** |
| dS279 | 96.63 | 94.14 | 76.75 |
| dS280 | 96.88 | 94.61 | 81.65 |
| dS281 | 95.40 | 91.47 | 74.04 |
| dS282 | 96.32 | 91.76 | 76.01 |
| dS283 | 92.40 | 83.92 | 46.69 |
| dS284 | 92.03 | 83.16 | NA |
| dS285 | 90.05 | 78.94 | -35.53 |

(continued)

| Test compound No. | 100 nM | 10 nM | 1 nM |
|---|---|---|---|
| | Average inhibition rate (%) | Average inhibition rate (%) | Average inhibition rate (%) |
| dS286 | 89.52 | 78.14 | 3.75 |
| dS287 | 91.46 | 84.14 | 74.14 |
| dS288 | 89.81 | 82.46 | 65.19 |

**Table 4.2 IC50 of dS319 for inhibition on target gene DGAT2 mRNA in primary human hepatocytes**

| Sequence | IC50 (pM) |
|---|---|
| dS319 | 62.3 |

**Example 4: Test for evaluating the in-vivo inhibitory activity of siRNA compounds on target gene DGAT2 in wild mice**

Animals

Male C57BL/6 mice, 6 weeks old, without particular pathogens, and raised in independent ventilation cages. Before the experiment, the animals were allowed to acclimate to the environment for 7 days.

Solvent and test compounds

Solvent: Phosphate buffer, used for preparing drug solutions on the day of administration.

Reagents and materials:

See the table below.

| Name of reagents and materials | Manufacturer | Article number |
|---|---|---|
| Trizol | Invitrogen | 15596018 |
| Stainless steel bead | Qiagen | 69989 |
| FastKing cDNA first strand synthesis kit (genome clean) | Qiagen | KR116-03 |
| GAPDH gene expression kit (Mm99999915_g1) | Thermo | 4351368 |

In-vivo experiment

The in-vivo experimental designs for the animal administration and the sample collection method were shown in the table below.

| Group | Number of animals in each group | Administration | | | | Time of liver dissection | Reading |
|---|---|---|---|---|---|---|---|
| | | Test agent | Dose (mg/kg) | Volume (mL/kg) | Schedule | | |
| 1 | 3 | PBS | / | 5 | | | |
| 3 | 3 | dS279 | 3 | 5 | | | |
| 4 | 3 | dS280 | 3 | 5 | | | |

(continued)

| Group | Number of animals in each group | Administration | | | | Time of liver dissection | Reading |
|---|---|---|---|---|---|---|---|
| | | **Test agent** | **Dose (mg/kg)** | **Volume (mL/kg)** | **Schedule** | | |
| 5 | 3 | dS281 | 3 | 5 | SC, day 0, once | Days 7 and 28: Collect liver treated with RNA-later | mRNA in liver (RT-qPCR) |
| 6 | 3 | dS282 | 3 | 5 | | | |
| 7 | 3 | dS283 | 3 | 5 | | | |
| 8 | 3 | dS284 | 3 | 5 | | | |
| 9 | 3 | dS285 | 3 | 5 | | | |
| 10 | 3 | dS286 | 3 | 5 | | | |
| 11 | 3 | dS287 | 3 | 5 | | | |

**Table 5 Test for evaluating the in-vivo inhibitory activity of siRNA compounds on target gene DGAT2 in wild mice 7 and 28 days after administration**

| siRNA No. | 7 days | 28 days |
|---|---|---|
| | **Average inhibition rate, %** | **Average inhibition rate, %** |
| dS279 | 67.1 | 60.5 |
| dS280 | 23.4 | 67.7 |
| dS281 | 36.7 | 56.6 |
| dS282 | 12.5 | 44.9 |
| dS283 | 12.9 | 21.4 |
| dS284 | -1.2 | 44.8 |
| dS285 | 32.1 | 48.9 |
| dS286 | 6.4 | 39.7 |
| dS287 | 55.9 | 49.0 |
| dS288 | -23.3 | -4.5 |

**Example 5: Activity evaluation of test compounds in mouse model receiving hydrodynamic injection of DGAT2 plasmid via tail vein**

**MATERIALS AND METHODS**

**MATERIAL**

**Animals**

BALB/c mice, female, 6-7 weeks old.

**DGAT2 plasmid DNA**

pcDNA-DGAT2 plasmid, having a concentration of 2 μg/μL, diluted with physiological saline for later use.

**Reagents and materials:**

See the table below.

| Name of reagents and materials | Manufacturer | Article number |
|---|---|---|
| Trizol | Invitrogen | 15596018 |
| Stainless steel bead | Qiagen | 69989 |
| FastKing cDNA first strand synthesis kit (genome clean) | Qiagen | KR116-03 |
| DGAT2 gene expression kit (Hs01045913_m1) | Thermo | 4351368 |

**TEST METHOD:**

**In-vivo experiment**

The hydrodynamic injection scheme of DGAT2 plasmid DNA via tail vein, the administration and the sample collection method were as follows.

**Treatment with compound:** The day of administration to mice was defined as day 0, the day before it was day -1, the day after it was day 1, and so on. On day 0, the mice were injected subcutaneously with 5 mL/kg of the solvent or the test compound, at a dosage of 3mpk.

**Hydrodynamic injection of DGAT2 plasmid DNA solution into mice via tail vein:** On day 4 or 14, the plasmid DNA was prepared with normal saline before injection, and stored at 4°C until use. All mice were injected with the plasmid DNA solution in a volume of 8% of the body weight (injection volume (mL)= body weight of mice (g) x 8%) in 5 seconds via the tail vein, and the weight of plasmid injected into each mouse was 10 μg.

**Endpoint of the experiment and collection of liver samples:** On day 5 or 15, that is, 24 hrs after the hydrodynamic injection of DGAT2 plasmid via the tail vein, all mice were euthanized by $CO_2$ inhalation. After euthanasia, blood samples were collected from the heart and liver samples were collected. 2 liver tissues of about 70 mg were immersed in RNA*later*®, and incubated overnight at 4 °C. The supernatant was discarded and the remainder was transferred to a freezer at -80°C, for mRNA detection later.

**Sample analysis**

**Quantitative PCR detection of DGAT2 gene expression in mouse liver**

RNA in the liver was extracted with Trizol. The method was briefly described as follows: 50-70 mg of liver tissue and 1.2 mL of Trizol were homogenized with Qiagen Tissue Lyser II at 26 Hz for 2 min and lyzed at room temperature for 5 min. 1 mL of the supernatant was taken, added to 200 uL of chloroform, vigorously shaken for 15 s, allowed to stand for 3 min, and centrifuged. The supernatant was collected, added with an equal volume of isopropanol, and stood overnight at -20°C for precipitation. The precipitate was washed twice with 70% , and dissolved in water without RNase. The concentration of the RNA sample was determined by Nanodrop ONE and diluted to 400 ng/uL with water without RNase for reverse transcription.

The experimental steps of reverse transcription could be made reference to the instruction of FastKing cDNA first strand synthesis kit (genome clean). Briefly, a gDNA removal mixture was prepared according to Table 6. The mixture (5 μL/sample) and 400 ng/μL RNA sample were added to a 96-well PCR reaction plate, incubated at 42°C for 3 min, and cooled on ice. A mixture for reverse transcription was prepared according to Table 7. The reverse transcription mixture (10 μL/sample) and the DNA removal product of the previous step were added to a 96-well PCR reaction plate, for reverse transcription. Reaction conditions: 42°C for 15 min; and 95°C for 3 min. The cDNA was stored at 4°C for further analysis.

**Table 6. Ingredients for gDNA-removal reaction**

| Ingredient | Volume for 1 reaction system (μL) |
|---|---|
| 5×gDNA | 2 |
| RNase-Free $ddH_2O$ | 3 |
| 400 ng/μL RNA sample | 5 |

**Table 7. Ingredients for RT-PCR reaction**

| Ingredient | Volume for 1 reaction system (μL) |
|---|---|
| 10×King RT Buffer | 2 |

(continued)

| Ingredient | Volume for 1 reaction system (μL) |
|---|---|
| FastKing RT Enzyme Mix | 1 |
| FQ-RT Primer Mix | 2 |
| RNase-Free dd$H_2O$ | 5 |

The method for detecting the expression levels of DGAT2 and NEO genes in mouse liver by quantitative PCR was briefly described as follows: As shown in Tables 8 and 9, a qPCR reaction mixture was prepared, and 2 μL of a cDNA sample diluted 4 times with RNase-free water was added for PCR reaction. Reaction conditions: 95°C for 10 min; and 40 cycles of 95°C for 15 s, and 60°C for 1 min.

**Table 8. Ingredients for qPCR Reaction (DGAT2)**

| Ingredients in qPCR reaction solution | Volume for 1 reaction system (μL) |
|---|---|
| Universal PCR Master Mix | 5 |
| Gene Expression Assay (DGAT2) | 1/6 |
| RNase-Free dd$H_2O$ | 17/6 |
| cDNA | 2 |

**Table 9. Ingredients for qPCR Reaction (NEO)**

| Ingredients in PCR reaction solution | Volume for 1 reaction system (μL) |
|---|---|
| Universal PCR Master Mix | 5 |
| Neo-probe | 0.2 |
| Neo-F | 0.4 |
| Neo-R | 0.4 |
| RNase-Free dd$H_2O$ | 2 |

The expression of the target gene in each sample was analyzed by relative quantification using ΔΔCt method. In this method, the Ct difference (ΔCt) between the target gene (DGAT2) and the internal reference gene (Neo) was determined, and the ΔCt value of the sample treated with the compound was compared with that of the control group.

The formula is:

$$\Delta Ct = \text{average Ct value of target gene- average Ct value of reference gene.}$$

ΔΔCt= ΔCt of the sample treated with the compound-the average ΔCt of the control group.

Gene expression level =$2^{-\Delta\Delta CT}$

**Data analysis:** The data are expressed as the average of each group of mouse samples ± standard error. Unless otherwise specified, n=4 or 5. Student's t test was used for statistical analysis.

**Table 10.1 Test for evaluating the in-vivo inhibitory activity of siRNA compounds on target gene DGAT2 in mice model receiving hDGAT2 HDI 5 days after administration**

| siRNA No. | Average inhibition rate, % **Day 1 after HDI (day 5 after administration)** |
|---|---|
| PBS | 0 |
| dS001 | 88.96890487 |
| dS279 | 94.9582804 |

(continued)

| siRNA No. | Average inhibition rate, % **Day 1 after HDI (day 5 after administration)** |
|---|---|
| dS280 | 96.6124228 |
| dS301 | 91.68046413 |
| dS302 | 97.4620214 |
| dS303 | 85.5258201 |
| dS304 | 66.33473761 |
| dS305 | 95.59400996 |
| dS306 | 96.71848804 |

**Table 10.2 Test for evaluating the in-vivo inhibitory activity of siRNA compounds on target gene DGAT2 in mice model receiving hDGAT2 HDI 15 days after administration**

| siRNA No. | Average inhibition rate, % **Day 1 after HDI (day 15 after administration)** |
|---|---|
| PBS | 0 |
| dS307 | 61.0289255 |
| dS301 | 78.88937944 |
| dS302 | 70.35349309 |
| dS319 | 91.78844845 |

**Example 6: Test of the inhibitory activity of the double-stranded ribonucleic acid conjugates of the present invention on the target gene DGAT2 in mice**

Animals: Male C57BL/6 mice, 6 weeks old, without particular pathogens. Before the experiment, the animals were allowed to acclimate to the environment for 7 days.

Solvent: phosphate buffer (PBS).

Reagents and materials: See the table below.

| Name of reagents and materials | Manufacturer | Article number |
|---|---|---|
| Trizol | Invitrogen | 15596018 |
| Stainless steel bead | Qiagen | 69989 |
| FastKing cDNA first strand synthesis kit (remove genome) | Qiagen | KR116-03 |
| GAPDH gene expression kit (Mm99999915_g1) | Thermo | 4351368 |

**In-vivo experiment:** The in-vivo experimental designs for the animal administration and the sample collection method were shown in the table below.

| Group | Number of animals in each group | **Administration** | | | | **Time of liver dissection** |
|---|---|---|---|---|---|---|
| | | **Test agent** | **Dose (mg/kg)** | **Volume (mL/kg)** | **Schedule** | |
| 1 | 3 | PBS | / | 5 | SC, On day 0, once | Day 7 |
| 2 | 3 | dS286 | 3 | 5 | | |
| 3 | 3 | dS307 | 3 | 5 | | |

Definition of days of experiment: The day of first administration to mice was defined as day 0, the day before it was day -1, the day after it was day 1, and so on.

All mice were given a single dose of PBS, the control compound or the test compound by SC on day 0. On day 7,

blood was taken from the submandibular vein plexus mice, and the plasma was collected. Then the mice were euthanized. Blood was taken from the heart for later use. The liver was collected for analysis of target mRNA level in the liver.

**Sample detection and analysis:** RT-PCR was used to quantitatively detect the level of target mRNA in liver tissue.

The expression of the target gene in each sample was analyzed by relative quantification using ΔΔCt method. In this method, the Ct difference (ΔCt) between the target gene (DGAT2) and the internal reference gene (GAPDH) was determined, and the ΔCt value of the sample treated with the compound was compared with that of the control group.

The formula is:

$$\Delta Ct = \text{average Ct value of target gene- average Ct value of reference gene.}$$

ΔΔCt= ΔCt of the sample treated with the compound-the average ΔCt of the control group.

$$\text{Gene expression level} = 2^{-\Delta\Delta CT}$$

Data analysis: The data are expressed as the average of each group of mouse samples ± standard error. Unless otherwise specified, n= 4. Student's t test was used for statistical analysis.

**Experimental results:** See Table 11.

**Table 11. Test results of the inhibitory activity of the double-stranded ribonucleic acid conjugates on the target gene DGAT2 in wild mice**

| Test agent | Average inhibition rate (%) (7 days) |
|---|---|
| dS286 | 86 |
| dS307 | 76 |

**Conclusions:** The double-stranded ribonucleic acid conjugates of the present invention effectively inhibit the expression of the target gene DGAT2.

**Example 7: Test of the inhibitory activity of the double-stranded ribonucleic acid conjugate on the target gene DGAT2 in mice receiving hydrodynamic injection of human DGAT2 plasmid via the tail vein**

**Animals:** BALB/c mice, female, 6-7 weeks old.

**DGAT2 plasmid DNA:** pcDNA-DGAT2 plasmid, having a concentration of 2 μg/μL, diluted with physiological saline for later use.

**Reagents and materials:** See the table below:

| Name of reagents and materials | Manufacturer | Article number |
|---|---|---|
| Trizol | Invitrogen | 15596018 |
| Stainless steel bead | Qiagen | 69989 |
| FastKing cDNA first strand synthesis kit (remove genome) | Qiagen | KR116-03 |
| DGAT2 gene expression kit (Hs01045913_m1) | Thermo | 4351368 |

**Experimental method:**

The in-vivo experimental designs for the hydrodynamic injection of DGAT2 plasmid DNA via the tail vein, the administration, and the sample collection method were shown in the table below.

Treatment with compound: The day of administration to mice was defined as day 0, the day before it was day -1,

the day after it was day 1, and so on. On day 0, the mice were injected subcutaneously with 5 mL/kg of the solvent or the test compound. The details were shown in Table 5.

Hydrodynamic injection of DGAT2 plasmid DNA solution into mice via tail vein: On day 14, the plasmid DNA was prepared with normal saline before injection, and stored at 4°C until use. All mice were injected with the plasmid DNA solution in a volume of 8% of the body weight (injection volume (mL)= body weight of mice (g) x 8%) in 5 seconds via the tail vein, and the weight of plasmid injected into each mouse was 10 μg.

Endpoint of the experiment and collection of liver samples: On day 15, that is, 24 hrs after the hydrodynamic injection of DGAT2 plasmid via the tail vein, all mice were euthanized by $CO_2$ inhalation. After euthanasia, blood samples were collected from the heart and liver samples were collected. 2 liver tissues of about 70 mg were immersed in RNA*later*®, and incubated overnight at 4°C. The supernatant was discarded and the remainder was transferred to a freezer at -80°C, for mRNA detection later.

| Group | Number of animals (animals/group) | HDI | Treatment with compound: | | | | Sample collection and test indexes |
|---|---|---|---|---|---|---|---|
| | | | Test Compound | Dose of administration (mg/kg) | Route and volume of administration | Administration regimen | |
| 1 | 5 | pcDNA-DGAT2, On day 3, 10 µg/animal | PBS | NA | Subcutaneous injection, 5 mL/kg | Single dose, day 0 | Liver sample: Day 15 (24 h after the injection of DGAT2 plasmid) Detection: DGAT2 mRNA by RT-qPCR |
| | 4 | | | | | | |
| 3 | 4 | | dS301 | 3 | | | |
| | 4 | | | | | | |
| | 4 | | | | | | |
| 6 | 4 | | dS316 | 3 | | | |

**Quantitative PCR detection of DGAT2 gene expression in mouse liver:**

RNA in the liver was extracted with Trizol. The concentration of the RNA sample was determined by Nanodrop ONE (ultra-micro ultraviolet-visible spectrophotometer) and diluted to 400 ng/uL with water without RNase for reverse transcription. Quantitative PCR detection of DGAT2 and NEO gene expressions in mouse liver:

**Experimental results:** See Table 6.

**Table 12. Test results of the inhibitory activity of the double-stranded ribonucleic acid conjugates on the target gene DGAT2 in mice**

| Test agent | Average inhibition rate, % (Day 15 after administration) |
|---|---|
| PBS | 0 |
| dS301 | 79 |
| dS316 | 66 |

**Conclusions:** The double-stranded ribonucleic acid conjugates of the present invention effectively inhibit the expression of the target gene DGAT2.

**Example 8: Test of the inhibitory activity of the double-stranded ribonucleic acid conjugate on the target gene DGAT2 in mice receiving hydrodynamic injection of human DGAT2 plasmid via the tail vein**

Animals: BALB/c mice, female, 6-7 weeks old.

DGAT2 plasmid DNA: pcDNA-DGAT2 plasmid, having a concentration of 2 μg/μL, diluted with physiological saline for later use.

Reagents and materials: See the table below.

| Name of reagents and materials | Manufacturer | Article number |
|---|---|---|
| Trizol | Invitrogen | 15596018 |
| Stainless steel bead | Qiagen | 69989 |
| FastKing cDNA first strand synthesis kit (remove genome) | Qiagen | KR116-03 |
| DGAT2 gene expression kit (Hs01045913_m1) | Thermo | 4351368 |

Experimental method: The in-vivo experimental designs for the hydrodynamic injection of DGAT2 plasmid DNA, the administration and the sample collection method were shown in the table below.

| Group | Number of animals (animals/group) | HDI | Treatment with compound: | | | | Sample collection and test indexes |
|---|---|---|---|---|---|---|---|
| | | | Test Compound | Dose of administration (mg/kg) | Route and volume of administration | Administration regimen | |
| 1 | 5 | | PBS | NA | | | |
| 2 | 4 | pcDNA-DGAT2, On day 3, 10 μg/animal | dS302 | 3 | Subcutaneous injection, 5 mL/kg | Single dose, day 0 | Liver sample: Day 15 (24 h after the injection of DGAT2 plasmid)<br>Detection: DGAT2 mRNA by RT-qPCR |

Treatment with compound: The day of administration to mice was defined as day 0, the day before it was day -1, the day after it was day 1, and so on. On day 0, the mice were injected subcutaneously with 5 mL/kg of the solvent or the test compound,

Hydrodynamic injection of DGAT2 plasmid DNA solution into mice via tail vein: On day 14, the plasmid DNA was prepared with normal saline before injection, and stored at 4°C until use. All mice were injected with the plasmid DNA solution in a volume of 8% of the body weight (injection volume (mL) = body weight of mice (g) x 8%) in 5 seconds via the tail vein, and the weight of plasmid injected into each mouse was 10 μg.

Endpoint of the experiment and collection of liver samples: On day 15, that is, 24 hrs after the hydrodynamic injection of DGAT2 plasmid via the tail vein, all mice were euthanized by $CO_2$ inhalation. After euthanasia, blood samples were collected from the heart and liver samples were collected. 2 liver tissues of about 70 mg were immersed in RNAlater®, and incubated overnight at 4 °C. The supernatant was discarded and the remainder was transferred to a freezer at -80°C, for mRNA detection later.

Quantitative PCR detection of DGAT2 gene expression in mouse liver: RNA in the liver was extracted with Trizol. The concentration of the RNA sample was determined by Nanodrop ONE (ultra-micro ultraviolet-visible spectrophotometer) and diluted to 400 ng/uL with water without RNase for reverse transcription. Quantitative PCR detection of DGAT2 and NEO gene expressions in mouse liver:

Experimental results: See Table 13.

**Table 13. Test results of the inhibitory activity of the double-stranded ribonucleic acid conjugates on the target gene DGAT2 in mice**

| Test agent | Average inhibition rate, % (Day 15 after administration) |
|---|---|
| PBS | 0 |
| dS302 | 70.4 |

Conclusions: The double-stranded ribonucleic acid conjugates of the present application effectively inhibit the expression of the target gene DGAT2.

**Example 9: Improvement of double-stranded ribonucleic acid conjugates on non-alcoholic steatohepatitis induced by a western diet in human DGAT2 carrying mice in in-vivo experiments**

**Materials and methods**

Humanized mice with B6-hDGAT2-TG target and non-alcoholic steatohepatitis model were provided by GemPharmatech LLC. On D0, subcutaneous administration and induction and feeding with western diet were started. On D29, the second injection was performed. Effective period: 8 weeks. 4 mice in one group were sacrificed at 4-week endpoint, and 4 mice in the other group were sacrificed at 8-week endpoint.

Plasma was collected every week to detect the levels of TG, Chol, HDL-C, LDL-C, ALT, and AST, and the liver was stained with HE and NASH score were obtained at the endpoint.

The test results are shown in Tables 14 and 15 and FIGs. 1 and 2.

**Table 14. Improvement of dS319 on NASH and fibrosis in non-alcoholic steatohepatitis induced by hDGAT2 combined with western diet at 4-week endpoint**

| Group | Grade of steatosis | Lobular inflammation | Ballooning degeneration | NAFLD score | Fibrosis score |
|---|---|---|---|---|---|
| B6-PBS | 0.0±0.0**** | 0.8±0.1**** | 0.0±0.0**** | 0.8±0.1**** | 0.0±0.0*** |
| B6-hDGAT2-PBS | 1.3±0.1 | 2.8±0.1 | 2.0±0.0 | 6.1±0.1 | 1.1±0.1 |
| B6-hDGAT2-dS319 3mpk | 0.7±0.1*** | 2.2±0.1** | 2.0±0.0 | 4.9±0.2*** | 0.5±0.1** |
| B6-hDGAT2-dS319 6mpk | 0.5±0.2**** | 1.6±0.2**** | 1.3±0.1**** | 3.4±0.2**** | 0.4±0.1*** |
| Note: The data is expressed as mean ± SEM, n=8, Vs G2,* : P<0.05; **: P<0.01; ***: P<0.001; ****: P<0.0001 | | | | | |

**Table 15. Improvement of dS319 on NASH and fibrosis in non-alcoholic steatohepatitis induced by hDGAT2 combined with western diet at 8-week endpoint**

| Group | Grade of steatosis | Lobular inflammation | Ballooning degeneration | NAFLD score | Fibrosis score |
|---|---|---|---|---|---|
| B6-PBS | 0.0±0.0**** | 0.9±0.2**** | 0.0±0.0**** | 0.9±0.2**** | 0.1±0.1**** |
| B6-hDGAT2-PBS | 2.0±0.0 | 3.0±0.0 | 2.0±0.0 | 7.0±0.0 | 1.9±0.1 |
| B6-hDGAT2-dS319 3mpk | 1.0±0.0**** | 1.8±0.2**** | 2.0±0.0 | 4.8±0.2**** | 0.9±0.1**** |
| B6-hDGAT2-dS319 6mpk | 1.1±0.1**** | 2.0±0.1**** | 1.3±0.1**** | 4.3±0.0**** | 0.8±0.2**** |

**Example 10: Test results of the inhibitory activity of the double-stranded ribonucleic acid conjugates on the target gene DGAT2 in non-naive Cynomolgus macaque**

The in-vivo experimental designs for subcutaneous injection in non-naive Cynomolgus macaque, the administration and the sample collection method were shown in the table below.

The day of administration to non-naive Cynomolgus macaque was defined as day 1, the day before it was day 0, the day after it was day 2, and so on. Each group had 2 animals. On days 1, 4, 8, 15, 22, 29, 36, 43, 50, and 57, the animals in Groups 1 and 2 were injected with the solvent or 4 mg/kg (0.5mL/kg) of the positive reference compound. On days 1 and 29, the animals in Groups 3 to 7 were injected subcutaneously with 0.5 mL/kg of the test compound. The details were shown in the table below.

| **Group** | **Gender/number** | **Treatment method** | | | | **Route of administration** |
|---|---|---|---|---|---|---|
| | **male** | **Test agent** | **Dose (mg/kg)** | **Volume of administration (mL/kg)** | **Vehicle** | |
| 1 | 2 | PBS | 0 | 0.5 | PBS (pH 7.4, 1×) | Subcutaneous injection |
| 4 | 2 | dS286 | 3 | 0.5 | PBS (pH 7.4, 1×) | Subcutaneous injection |
| 5 | 2 | dS286 | 10 | 0.5 | PBS (pH 7.4, 1×) | Subcutaneous injection |
| 6 | 2 | dS319 | 3 | 0.5 | PBS (pH 7.4, 1×) | Subcutaneous injection |
| 7 | 2 | dS319 | 10 | 0.5 | PBS (pH 7.4, 1×) | Subcutaneous injection |

On day 0 (0 h) before administration by SC, and days 7, 15, 28, 57, and 99 after administration, the liver sample was collected. Collection method: The animals was fasted for 12-16 hrs before sample collection. Collection of liver biopsy tissue: The collection process would be operated and recorded according to the SOP of the research institution. The location of the liver was determined by B ultrasound scan, the right liver was mainly used, and the gallbladder and major blood vessels were avoided. The biopsy needle was inserted under the guidance of the B-ultrasound probe. Guided by the image on the B-ultrasound screen, the needle entered the liver away from the gallbladder and major blood vessels, and the materials were sampled by puncture. After biopsy, the biopsy needle was withdrawn and the samples were collected. The obtained samples were immediately immersed in PBS with RNA*later*®, and allowed to stand overnight at 4°C. Then the residual liquid was sucked off, and the remainder was stored at -80°C or below until it was transported in the presence of dry ice and detected.

Quantitative PCR detection of DGAT2 gene expression in the liver of Cynomolgus macaque:

Trizol was homogenized by Qiagen Tissue Lyser II at 26 Hz for 2 min and lyzed at room temperature for 5 min. 1 mL of the supernatant was taken, added to 200 uL of chloroform, shaken for 15 s, allowed to stand for 3 min, and centrifuged. The supernatant was collected, added with an equal volume of isopropanol, and stood overnight at -20°C for precipitation. The precipitate was washed twice with 70%, and dissolved in water without RNase. The concentration of the

RNA sample was determined by Nanodrop ONE and diluted to 200 ng/μL with water without RNase for reverse transcription.

The experimental steps of reverse transcription could be made reference to the instruction of HiScript® III RT SuperMix for qPCR (+gDNA wiper). Briefly, a gDNA removal mixture was prepared according to the table below. A mixture (11 μL/sample) of 4 x gDNA wiper Mix and RNase-Free $ddH_2O$ and 1000 ng RNA sample (5 μL RNA sample with a concentration of 200 ng/μL) were added to a 96-well PCR reaction plate, incubated at 42°C for 2 min, and cooled on ice. 4 μL 5 x HiScript III qRT SuperMix and the DNA removed product from the previous step were added to a 96-well PCR reaction plate for reverse transcription. Reaction conditions: 37°C for 15 min; and 85°C for 5 sec. The cDNA was stored at 4°C for further analysis.

**Ingredients for gDNA-removal reaction**

| Ingredient | Volume for 1 reaction system (μL) |
|---|---|
| 4 × gDNA wiper Mix | 4 |
| 200 ng/μL RNA sample | 5 |
| RNase-Free $ddH_2O$ | 7 |

The method for detecting the expression levels of DGAT2 and GAPDH genes in the liver of Cynomolgus macaque by quantitative PCR was briefly described as follows: As shown in the table below, a qPCR reaction mixture was prepared, and 2 μL of a cDNA sample diluted 2 times with RNase-free water was added for PCR reaction. Reaction conditions: 95°C for 10 min; and 40 cycles of 95°C for 15 sec, and 60°C for 1 min.

**Ingredients for qPCR reaction (DGAT2)**

| Ingredients in PCR reaction solution | Volume for 1 reaction system (μL) |
|---|---|
| Universal PCR Master Mix | 5 |
| DGAT2-probe | 0.2 |
| DGAT2-F | 0.4 |
| DGAT2-R | 0.4 |
| RNase-Free ddH2O | 2 |

**Experimental results:** See Table 16.

**Table 16. Test results of the inhibitory activity of the double-stranded ribonucleic acid conjugates on the target gene DGAT2 in non-naive Cynomolgus macaque**

| Test agent | Dose | Average inhibition rate, % (D0 before administration) | Average inhibition rate, % (D28) | Average inhibition rate, % (D56) | Average inhibition rate, % (D99) |
|---|---|---|---|---|---|
| PBS | 0 | 0 | 29.6 | -12.3 | 41.4 |
| dS286 | 3mpk | 0 | 54.6 | 55.8 | 27.3 |
| dS286 | 10mpk | 0 | 65.4 | 34.8 | 46.3 |
| dS319 | 3mpk | 0 | 86.6 | 91.1 | 72.2 |
| dS319 | 10mpk | 0 | 96.0 | 97.9 | 89.1 |

All values were normalized to the average of the group on day 0.

**Example 11: Test results of the inhibitory activity of dS319 on the target gene DGAT2 in non-naive Cynomolgus macaque**

**In-vivo experiment:** The in-vivo experimental designs for subcutaneous injection in non-naive Cynomolgus macaque, the administration and the sample collection method were shown in the table below. The day of administration to non-naive Cynomolgus macaque was defined as day 1, the day before it was day 0, the day after it was day 2, and so on. Each group had 2 animals. On days 1 and 29, the animals were injected subcutaneously with the solvent or 0.5 mL/kg of the test compound.

| Group | Gender/number | Treatment method | | | | | Route of administration |
|---|---|---|---|---|---|---|---|
| | male | Test agent | Dose | Concentration | Volume of administration | Vehicle | |
| | | | (mg/kg) | (mg/mL) | (mL/kg) | | |
| 1 | 2 | PBS | 0 | 0 | 0.5 | PBS (pH 7.4, 1×) | Subcutaneous injection |
| 2 | 2 | dS319 | 3 | 6 | 0.5 | PBS (pH 7.4, 1×) | Subcutaneous injection |

On day 0 (0 h) before administration by SC, and days 7, 29, 56 and 99 after administration, the liver sample was collected. Collection method: The animals was fasted for 12-16 hrs before sample collection. Collection of liver biopsy tissue: The collection process would be operated and recorded according to the SOP of the research institution.

**Experimental results:** See Table 17.

**Table 17. Test results of the inhibitory activity of the double-stranded ribonucleic acid conjugates on the target gene DGAT2 in non-naive Cynomolgus macaque**

| Group | Test agent | Dose | Average inhibition rate, % | | | | |
|---|---|---|---|---|---|---|---|
| | | | D0 before administration | D7 | D28 | D56 | D99 |
| 1 | PBS | 0 | 0 | -44.4 | -33.7 | -84.5 | -28 |
| 2 | dS319 | 3mpk | 0 | 84.9 | 93.2 | 89.4 | 88 |

**Conclusions:** The double-stranded ribonucleic acid conjugate dS319 has a long-term inhibitory activity on the target gene DGAT2 in non-naive Cynomolgus macaque, and is superior to clinically used molecules with respect to the dosage and injection frequency.

## Claims

1. An siRNA, comprising a sense strand and an anti-sense strand, at least part of the sense strand and at least part of the anti-sense strand being reversely complementary, to form a duplex region, wherein the sense strand comprises 17 to 22 consecutive bases of any one of the sequences set forth in SEQ ID NOs: 1-96; further, comprises 20 consecutive bases of any one of the sequences set forth in SEQ ID NOs: 1-96; and further, comprises a base sequence as set forth in any one of SEQ ID NOs: 1-96.

2. An siRNA, comprising a sense strand and an anti-sense strand, at least part of the sense strand and at least part of the anti-sense strand being reversely complementary, to form a duplex region, wherein the anti-sense strand comprises 17 to 22 consecutive bases of any one of the sequences set forth in SEQ ID NOs: 97-192; further, comprises 20 consecutive bases of any one of the sequences set forth in SEQ ID NOs: 97-192; and further, comprises a base sequence as set forth in any one of SEQ ID NOs: 97-192 and 194-199.

3. The siRNA according to claim 1 or 2, wherein

a) the sense strand comprises 17 to 22 (and further 20) consecutive bases of a sequence as set forth in SEQ ID

NO: 77 or SEQ ID NO: 201, and the anti-sense strand comprises 17 to 22 (and further 20) consecutive bases of a sequence as set forth in SEQ ID NO: 173, 194, 195 or 200; or
b) the sense strand comprises 17 to 22 (and further 20) consecutive bases of a sequence as set forth in SEQ ID NO: 17, and the anti-sense strand comprises 17 to 22 (and further 20) consecutive bases of a sequence as set forth in SEQ ID NO: 113, 196, or 197; or
c) the sense strand comprises 17 to 22 (and further 20) consecutive bases of a sequence as set forth in SEQ ID NO: 80, and the anti-sense strand comprises 17 to 22 (and further 20) consecutive bases of a sequence as set forth in SEQ ID NO: 176, 198 or 199.

**4.** The siRNA according to claim 3, wherein

a) the sense strand comprises a sequence as set forth in SEQ ID NO: 77 or SEQ ID NO: 201, and the anti-sense strand comprises a sequence as set forth in SEQ ID NO: 194, SEQ ID NO: 195 or SEQ ID NO: 200; or
b) the sense strand comprises a sequence as set forth in SEQ ID NO: 17, and the anti-sense strand comprises a sequence as set forth in SEQ ID NO: 196 or SEQ ID NO: 197; or
c) the sense strand comprises a sequence as set forth in SEQ ID NO: 80, and the anti-sense strand comprises a sequence as set forth in SEQ ID NO: 198 or SEQ ID NO: 199.

**5.** The siRNA according to any one of claims 1 to 4, wherein the siRNA comprises one or more modified nucleotide(s); and further, all the nucleotides are modified nucleotides.

**6.** The siRNA according to claim 5, wherein the modified nucleotide is selected from a nucleotide modified with 2'-methoxy or a nucleotide modified with 2'-fluoro.

**7.** The siRNA according to any one of claims 1 to 6, wherein the siRNA comprises an abasic nucleotide; and optionally, the abasic nucleotide is an inverted abasic nucleotide.

**8.** The siRNA according to claim 7, wherein the sense strand of the siRNA comprises the abasic nucleotide at the 5' end.

**9.** The siRNA according to any one of claims 1 to 8, wherein the siRNA comprises one or more modified internucleoside linkages.

**10.** The siRNA according to claim 9, wherein the modified internucleoside linkage is a phosphorothioate linkage.

**11.** The siRNA according to any one of claims 1 to 10, wherein the siRNA comprises a 5'-terminal phosphate modification or a phosphate analog modification; and further, the anti-sense strand of the siRNA comprises a 5'-terminal with phosphate modification or a phosphate analog modification at the 5' end.

**12.** The siRNA according to any one of claims 1 to 11, wherein the phosphate analog modification is a vinyl phosphonate (VP) modification.

**13.** The siRNA according to claim 1 or 2, wherein the siRNA is selected from dS004 - dS099.

**14.** The siRNA according to claim 1 or 2, wherein the siRNA is selected from any one of siRNA set forth in Table 2; and further, the siRNA is selected from dS004, dS005, dS006, dS007, dS008, dS009, dS010, dS011, dS012, dS013, dS014, dS015, dS016, dS017, dS018, dS019, dS020, dS021, dS022, dS023, dS024, dS025, dS026, dS027, dS028, dS029, dS030, dS031, dS032, dS033, dS034, dS035, dS036, dS037, dS038, dS039, dS040, dS041, dS042, dS043, dS044, dS045, dS046, dS047, dS048, dS049, dS050, dS051, dS052, dS053, dS054, dS055, dS056, dS057, dS058, dS059, dS060, dS061, dS062, dS063, dS064, dS065, dS066, dS067, dS068, dS069, dS070, dS071, dS072, dS073, dS074, dS075, dS076, dS077, dS078, dS079, dS080, dS081, dS082, dS083, dS084, dS085, dS086, dS087, dS088, dS089, dS090, dS091, dS092, dS093, dS094, dS095, dS096, dS097, dS098, dS099, dS279, dS280, dS281, dS282, dS283, dS284, dS285, dS286, dS287 or dS288.

**15.** An siRNA conjugate, comprising the siRNA according to any one of claims 1 to 14 and a ligand group, wherein the ligand group is conjugated to the siRNA, the ligand group comprises 1, 2, 3 or 4 GalNAc groups, and the number of the ligand group is 1, 2, 3, 4, 5, 6 or 7.

**16.** The siRNA conjugate according to claim 15, wherein the ligand group is conjugated at the 3' end of the sense strand.

**17.** The siRNA conjugate according to claim 15 or 16, wherein the siRNA conjugate is selected from any one of siRNA conjugate shown in Table 2; and further, the siRNA conjugate is selected from dS301, dS302, dS303, dS304, dS305, dS306, dS307, dS316, dS319 or dS324.

**18.** The siRNA conjugate according to any one of claims 15 to 17, wherein the ligand group is selected from NAG37 or L96.

**19.** A pharmaceutical composition, comprising the siRNA according to any one of claims 1 to 14 or the siRNA conjugate according to any one of claims 15 to 18, and a pharmaceutically acceptable carrier.

**20.** Use of the siRNA according to any one of claims 1 to 14 or the siRNA conjugate according to any one of claims 15 to 18 in the manufacture of a medicament for treating diseases related to the expression of DGAT2, wherein further, the diseases related to the expression of DGAT2 are lipid metabolism diseases; and still further, the diseases related to the expression of DGAT2 are non-alcoholic fatty liver disease and non-alcoholic steatohepatitis.

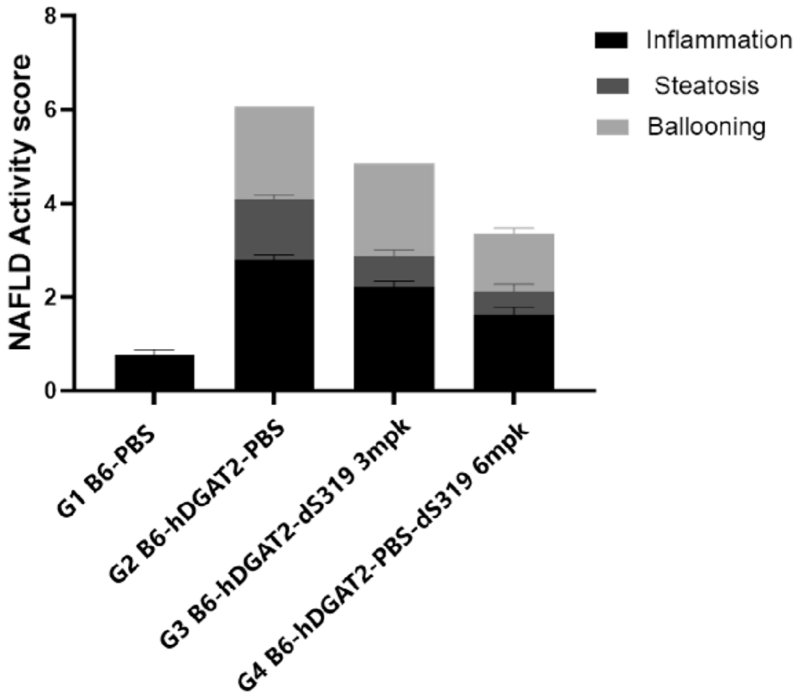


Improvement on non-alcoholic steatohepatitis (NASH) induced by hDGAT2 combined with western diet by NAFLD activity score x dS319 over 4 weeks

FIG. 1

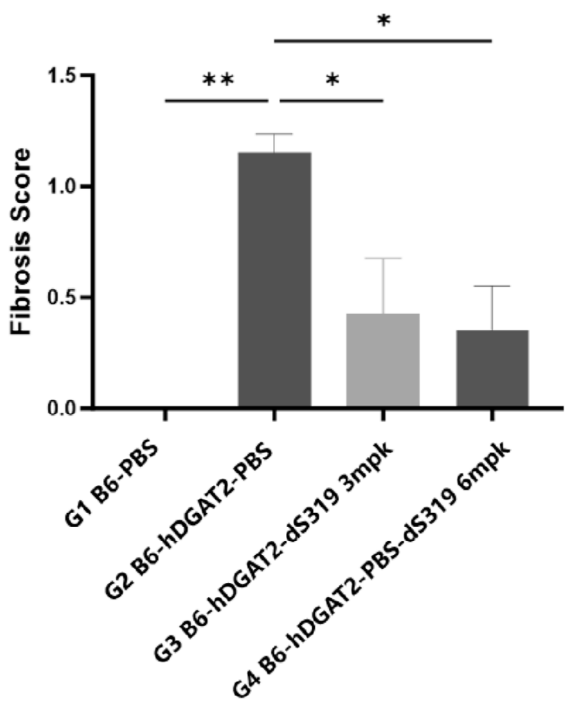


Improvement on fibrosis in non-alcoholic steatohepatitis induced by hDGAT2 combined with western diet by NAFLD activity score x dS319 over 4 weeks

FIG. 2

## INTERNATIONAL SEARCH REPORT

International application No.
**PCT/CN2024/095285**

**A. CLASSIFICATION OF SUBJECT MATTER**

C12N15/113(2010.01)i; A61P1/16(2006.01)i; A61K31/713(2006.01)i; A61K47/54(2017.01)i; A61K31/712(2006.01)i; A61P3/06(2006.01)i

According to International Patent Classification (IPC) or to both national classification and IPC

**B. FIELDS SEARCHED**

Minimum documentation searched (classification system followed by classification symbols)

IPC: C12N; A61P; A61K

Documentation searched other than minimum documentation to the extent that such documents are included in the fields searched

Electronic data base consulted during the international search (name of data base and, where practicable, search terms used)

CNABS, VEN, CNKI, CNTXT, USTXT, EPTXT, PUBMED, WEB OF SCIENCE, NCBI, STNext, 中国生物序列检索系统, China Biological Sequence Search System: 二酰甘油酰基转移酶2, 双甘油酯酰基转移酶2, 干扰RNA, 干涉RNA, 脂质代谢, DGAT, DGAT2, dsRNA, NAFLD, NASH, 非酒精性脂肪性肝病, 非酒精性脂肪性肝炎, shrna, siRNA, SEQ ID Nos: 1 and 97, 维亚臻, 何琼尔, 王明杰, 邹晓明, 杨宏宽

**C. DOCUMENTS CONSIDERED TO BE RELEVANT**

| Category* | Citation of document, with indication, where appropriate, of the relevant passages | Relevant to claim No. |
|---|---|---|
| X | WO 2023041508 A2 (ARGONAUTE RNA LIMITED) 23 March 2023 (2023-03-23)<br>description, page 3, paragraphs 20-29, page 4, paragraphs 4-17, page 6, paragraphs 21-22, page 7, paragraphs 5-9, page 11, paragraphs 25-36, page 12, paragraphs 2-4, page 17, paragraphs 10-14 and 30-36, and pages 27-28, table 3 | 1-2, 5-16, 18-20 |
| X | WO 2022136466 A1 (ARGONAUTE RNA LIMITED) 30 June 2022 (2022-06-30)<br>pp. 25-26, table 3 | 1-2, 5-16, 18-20 |
| A | CN 110520531 A (SILENCE THERAPEUTICS GMBH) 29 November 2019 (2019-11-29)<br>embodiment 10 | 1-2, 5-16, 18-20 |
| A | US 2022228141 A1 (UNIVERSITY OF MASSACHUSETTS) 21 July 2022 (2022-07-21)<br>description, embodiment 1 | 1-2, 5-16, 18-20 |
| A | WO 2022031850 A2 (DICERNA PHARMACEUTICALS INC.) 10 February 2022 (2022-02-10)<br>claims 1-35 | 1-2, 5-16, 18-20 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **26 August 2024** | **02 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/095285**

**C. DOCUMENTS CONSIDERED TO BE RELEVANT**

| Category* | Citation of document, with indication, where appropriate, of the relevant passages | Relevant to claim No. |
|---|---|---|
| A | CN 109477106 A (IONIS PHARMACEUTICALS, INC.) 15 March 2019 (2019-03-15) claims 1-57 | 1-2, 5-16, 18-20 |

Form PCT/ISA/210 (second sheet) (July 2022)

**INTERNATIONAL SEARCH REPORT**

International application No.
**PCT/CN2024/095285**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**

International application No.
**PCT/CN2024/095285**

**Box No. III Observations where unity of invention is lacking (Continuation of item 3 of first sheet)**

This International Searching Authority found multiple inventions in this international application, as follows:

Group 1: claims 1-2 (in part), 5-16 (in part) and 18-20 (in part) relate to siRNA, a sense strand of which contains 17 to 22 consecutive bases of SEQ ID No. 1 and an antisense strand of which contains 17 to 22 consecutive bases of SEQ ID No. 97; and an siRNA conjugate, a pharmaceutical composition, and the use thereof in the preparation of a drug for treating diseases related to DGAT2 expression.

Group 2: claims 1-2 (in part), 5-16 (in part) and 18-20 (in part) relate to siRNA, a sense strand of which contains 17 to 22 consecutive bases of SEQ ID No. 2 and an antisense strand of which contains 17 to 22 consecutive bases of SEQ ID No. 98; and an siRNA conjugate, a pharmaceutical composition, and the use thereof in the preparation of a drug for treating diseases related to DGAT2 expression.

...

Group 77: claims 1-20 (in part) relate to siRNA, a sense strand of which contains 17 to 22 consecutive bases of SEQ ID No. 77 and an antisense strand of which contains 17 to 22 consecutive bases of SEQ ID No. 173; and an siRNA conjugate, a pharmaceutical composition, and the use thereof in the preparation of a drug for treating diseases related to DGAT2 expression.

...

Group 96: claims 1-2 (in part), 5-16 (in part) and 18-20 (in part) relate to siRNA, a sense strand of which contains 17 to 22 consecutive bases of SEQ ID No. 96 and an antisense strand of which contains 17 to 22 consecutive bases of SEQ ID No. 192; and an siRNA conjugate, a pharmaceutical composition, and the use thereof in the preparation of a drug for treating diseases related to DGAT2 expression.

The same or corresponding technical feature among the inventions in groups 1-96 described above is: siRNA targeting DGAT2. However, the prior art discloses an siRNA duplex involved for DGAT2 (see US 2022228141 A1, description, embodiment 1, publication date: 21 July 2022). Therefore, the same or corresponding technical feature among the above 96 groups of inventions cannot be used as a special technical feature that defines a contribution which the inventions make over the prior art; thus, the inventions do not belong to a single general inventive concept, lack unity of invention, and do not comply with PCT Rule 13.1.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-2 (in part), 5-16 (in part), 18-20 (in part)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/CN2024/095285**

| Patent document cited in search report | Publication date (day/month/year) | Patent family member(s) | Publication date (day/month/year) |
|---|---|---|---|
| WO 2023041508 A2 | 23 March 2023 | CA 3229020 A1 | 23 March 2023 |
| | | WO 2023041508 A3 | 08 June 2023 |
| | | EP 4402263 A2 | 24 July 2024 |
| WO 2022136466 A1 | 30 June 2022 | EP 4237561 A1 | 06 September 2023 |
| | | JP 2024500035 A | 04 January 2024 |
| CN 110520531 A | 29 November 2019 | US 2022090067 A1 | 24 March 2022 |
| | | US 2020095580 A1 | 26 March 2020 |
| | | US 11015198 B2 | 25 May 2021 |
| | | JP 2022173554 A | 18 November 2022 |
| | | AU 2018247925 A1 | 03 October 2019 |
| | | AU 2018247925 B2 | 06 June 2024 |
| | | RS 63836 B1 | 31 January 2023 |
| | | SI 3607069 T1 | 28 February 2023 |
| | | EP 4219713 A2 | 02 August 2023 |
| | | EP 4219713 A3 | 16 August 2023 |
| | | CA 3057565 A1 | 11 October 2018 |
| | | HRP 20221400 T1 | 06 January 2023 |
| | | LT 3607069 T | 10 January 2023 |
| | | EP 3607069 A1 | 12 February 2020 |
| | | EP 3607069 B1 | 02 November 2022 |
| | | DK 3607069 T3 | 21 November 2022 |
| | | PL 3607069 T3 | 06 March 2023 |
| | | ES 2932831 T3 | 26 January 2023 |
| | | JP 2020516312 A | 11 June 2020 |
| | | JP 7155239 B2 | 18 October 2022 |
| | | HUE 061247 T2 | 28 June 2023 |
| | | IL 268828 A | 31 October 2019 |
| | | WO 2018185241 A1 | 11 October 2018 |
| | | FI 3607069 T3 | 13 January 2023 |
| US 2022228141 A1 | 21 July 2022 | AU 2021382621 A1 | 22 June 2023 |
| | | AU 2021382621 A9 | 08 February 2024 |
| | | EP 4247391 A1 | 27 September 2023 |
| | | WO 2022109398 A1 | 27 May 2022 |
| | | WO 2022109398 A9 | 29 September 2022 |
| | | JP 2023550485 A | 01 December 2023 |
| WO 2022031850 A2 | 10 February 2022 | EP 4192953 A2 | 14 June 2023 |
| | | IL 300299 A | 01 April 2023 |
| | | TW 202221120 A | 01 June 2022 |
| | | CA 3190594 A1 | 10 February 2022 |
| | | CL 2023000367 A1 | 06 October 2023 |
| | | US 2023340487 A1 | 26 October 2023 |
| | | AU 2021320219 A1 | 02 March 2023 |
| | | JP 2023538283 A | 07 September 2023 |
| | | KR 20230047453 A | 07 April 2023 |
| | | MX 2023001451 A | 23 March 2023 |
| | | WO 2022031850 A3 | 14 April 2022 |
| | | BR 112023001820 A2 | 28 March 2023 |
| CN 109477106 A | 15 March 2019 | CL 2018000070 A1 | 27 July 2018 |
| | | AU 2016294347 A1 | 04 January 2018 |

Form PCT/ISA/210 (patent family annex) (July 2022)

**INTERNATIONAL SEARCH REPORT**
**Information on patent family members**

International application No.
**PCT/CN2024/095285**

| Patent document cited in search report | Publication date (day/month/year) | Patent family member(s) | | | Publication date (day/month/year) |
|---|---|---|---|---|---|
| | | AU | 2016294347 | B2 | 28 July 2022 |
| | | MX | 2018000412 | A | 14 March 2018 |
| | | BR | 112017028194 | A2 | 06 November 2018 |
| | | BR | 112017028194 | B1 | 14 March 2023 |
| | | US | 2020040341 | A1 | 06 February 2020 |
| | | US | 11312962 | B2 | 26 April 2022 |
| | | JP | 2022106727 | A | 20 July 2022 |
| | | MY | 192997 | A | 20 September 2022 |
| | | KR | 20180027522 | A | 14 March 2018 |
| | | KR | 102640776 | B1 | 23 February 2024 |
| | | JP | 2024084827 | A | 25 June 2024 |
| | | DK | 3320094 | T3 | 23 May 2022 |
| | | WO | 2017011276 | A1 | 19 January 2017 |
| | | ZA | 201708453 | B | 30 August 2023 |
| | | JP | 2018525030 | A | 06 September 2018 |
| | | JP | 7054672 | B2 | 14 April 2022 |
| | | IL | 256622 | A | 28 February 2018 |
| | | IL | 256622 | B1 | 01 May 2023 |
| | | IL | 256622 | B2 | 01 September 2023 |
| | | ES | 2917181 | T3 | 07 July 2022 |
| | | EP | 3320094 | A1 | 16 May 2018 |
| | | EP | 3320094 | A4 | 10 April 2019 |
| | | EP | 3320094 | B1 | 13 April 2022 |
| | | US | 2023047452 | A1 | 16 February 2023 |
| | | PE | 20180800 | A1 | 09 May 2018 |
| | | SG | 10202006563 | PA | 28 August 2020 |
| | | US | 2018312845 | A1 | 01 November 2018 |
| | | CA | 2991894 | A1 | 19 January 2017 |
| | | CO | 2017013065 | A2 | 28 March 2018 |
| | | EP | 4092119 | A2 | 23 November 2022 |
| | | EP | 4092119 | A3 | 22 March 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MARCHESINI G et al.** *Hepatology*, 2003, vol. 37, 917-923
- **BYRNE CD** ; **TARGHER G.** *J Hepatol*, April 2015, vol. 62 (1S), S47-S64
- **FARRELL GC** ; **LARTER CZ**. *Hepatology*, 2006, vol. 43, S99-S112
- **COHEN JC et al.** *Science*, 2011, vol. 332, 1519-1523
- **CASES S et al.** *Proc Natl Acad Sci USA*, 1998, vol. 95, 13018-13023
- **CASES S et al.** *J Biol Chem*, 2001, vol. 276, 38870-38876
- **CAO J et al.** *J Lipid Res*, 2007, vol. 48, 583-591
- **CHENG D et al.** *J Biol Chem*, 2008, vol. 283, 29802-29811
- **STONE SJ et al.** *J Biol Chem*, 2004, vol. 279, 11767-11776
- **MEEGALLA RL et al.** *Biochem Biophys Res Commun*, 2002, vol. 298, 317-323
- **YU XX et al.** *Hepatology*, 2005, vol. 42, 362-371
- **YAMAGUCHI K et al.** *HEPATOLOGY*, 2007, vol. 45 (6), 1366-1374
- **YENILMEZ, B et al.** *Molecular Therapy*, March 2022, vol. 30 (3)